# EUROPEAN PATENT APPLICATION

(11) **EP 0 861 831 A1**
(43) Date of publication of application: **02.09.1998**
(21) Application number: 96931252.9
(22) Date of filing: 19.09.1996
(51) Int. Cl.: C07D 207/327, C07D 207/337, C07D 209/08, C07D 209/18, C07D 213/56, C07D 233/61, C07D 233/64, C07D 249/18, C07D 263/14, C07D 277/30, C07D 277/44

(54) **HETEROARYL-SUBSTITUTED ACRYLOYLGUANIDINE DERIVATIVES AND MEDICINAL COMPOSITION THEREOF**

(30) Priority: 20.09.1995 JP 241716/95
(71) Applicant: YAMANOUCHI PHARMACEUTICAL CO., LTD., Tokyo 103 (JP)
(72) Inventor: KIKUCHI, Kazumi, Tokyo 120 (JP); TOYOSHIMA, Akira, Tsukuba-shi, Ibaraki 305 (JP); OKAZAKI, Toshio, Ryugasaki-shi, Ibaraki 301 (JP); TAKANASHI, Masahiro, Kashiwa-shi, Chiba 277 (JP); YANAGISAWA, Isao, Tokyo 177 (JP)
(74) Representative: Geering, Keith Edwin
(86) International application number: JP9602696
(87) International publication number: WO9711055

(57) **Abstract**

Disclosed are heteroaryl-substituted acryloylguanidine derivatives of formula (I) and their salts. 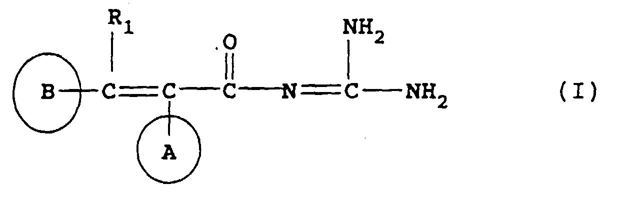 wherein ring A represents a 5-membered or 6-membered heteroaryl group which may optionally be substituted and may optionally be condensed with one or two benzene rings; ring B represents an optionally-substituted aryl group; and R₁ represents a hydrogen atom, a halogen atom, or a lower alkyl group optionally substituted by one or more halogen atoms. As acting as an Na⁺/H⁺ exchange inhibitor, the derivatives and their salts are useful in medicine, especially in prevention, treatment and diagnosis of various disorders to be caused by the Na⁺/H⁺ exchanger, such as hypertension, arrhythmia, angina pectoris, arteriosclerosis, diabetic complications, etc.

## Description

### TECHNICAL FIELD

The present invention relates to medicaments, especially to heteroaryl-substituted acryloylguanidine derivatives and their salts that inhibit the action of the Na⁺/H⁺ exchange.

### BACKGROUND ART

Various intracelluar reactions are influenced by pH in cells, and the H⁺ concentration gradient in cells produces the motive force for the synthesis of ATP therein. Therefore, in order to make cells exhibit their normal functions, the pH in cells must be controlled normally. The Na⁺/H⁺ exchanger exhibits some functions of controlling pH in cells. Recently, it has been clarified that the Na⁺/H⁺ exchanger has close relation to the activation of cells by various physiologically-active substances. In addition, it has been found and specifically noted that the promoted activation of the Na⁺/H⁺ exchanger has relation to the presentation, the continuation and even the worsening of the symptoms of some diseases. For example, various reports have heretofore been disclosed, referring to the relationship between the activities of the Na⁺/H⁺ exchanger and the symptoms of various diseases and physiological activities, such as ischemic-reperfusion myocardial injury and reperfusion arrhythmia (see Scholtz, W., et al., Br. J. Pharmacol., 109, 562 (1993)), hypertension (see Rosskof, D., et al., Hypertens. 21, 607 (1993)), cardiac hypertrophy (see de la Sierra, A., et al., Circulation, 88, 1628 (1993)), vascular hypertrophy (see Kranzhofer, R., et al., Circ. Res. 73, 246 (1993)), diabetic complications (see Canessa, M., et al., Hypertens. 11, 823 (1993)), bronchoconstriction by endothelin (see Battistini, B., et al., Biochem. Biophys. Res. Commun., 175, 583 (1991)), glutamic acid-induced death of neurocytes (see Manev, H., et al., Neuropharmacol., 29, 1103 (1990)), and bone resorption (see Hall, T. J., et al., Biochem. Biophys. Res. Commun., 188, 1097 (1992)).

As an example of the Na⁺/H⁺ exchange inhibitors, there has been known for a long period of time amiloride which is a kind of K⁺-retaining diuretics. It has been reported that amiloride has an antiarrhythmic action (see Mol. Pharmacol., 25, 131-136 (1984)). However, amiloride is problematic in the specificity of its action. Amiloride has such an antiarrhythmic action but has a blood pressure depressing action and also a salt-excreting action. The latter two are unfavorable side actions in treating arrhythmia with amiloride.

On the other hand, it has been reported that amiloride derivatives have an activity of inhibiting the Na⁺/H⁺ exchange and an antiarrhythmic action (for example, see J. Membrane Biol., 105, 1-21 (1988)). Recently, in addition, it has been reported that benzoylguanidine derivatives have an activity of inhibiting the Na⁺/H⁺ exchange and an antiarrhythmic action (for example, see Japanese Patent Application Laid-Open No. 3-106858).

Of acryloylguanidine derivatives, a diphenyl-substituted acryloylguanidine derivative of the following formula has been reported in a recent publication (see Am. J. Physiol., 260, C271 (1991)). In the publication, it is said that the compound has a low reactivity with antiamiloride antibodies and is therefore unsatisfactory as an epitope for amiloride. However, there is no disclosure in the publication relating to not only the the Na⁺/H⁺ exchange-inhibiting activity of the compound but also the use of the compound in medicine.

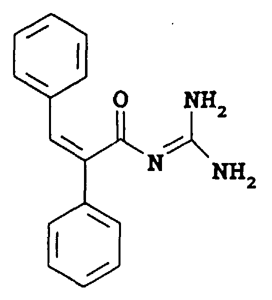

### DISCLOSURE OF THE INVENTION

We, the inventors of the present invention have assiduously studied various compounds which are expected to have an Na⁺/H⁺ exchange-inhibiting activity and, as a result, have found that acryloylguanidine derivatives having a heteroaryl substituent at the 2-position have a good Na⁺/H⁺ exchange-inhibiting activity and are useful in prevention; treatment and diagnosis of disorders to be caused by the Na⁺/H⁺ exchange. On the basis of these findings, we have completed the present invention.

Specifically, the present invention relates to heteroaryl-substituted acryloylguanidine derivatives of the following general formula (I) and their salts. 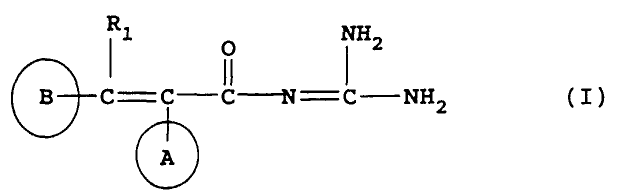 wherein ring A represents a 5-membered or 6-membered heteroaryl group which may optionally be substituted and may optionally be condensed with one or two benzene rings; ring B represents an optionally-substituted aryl group; and R₁ represents a hydrogen atom, a halogen atom, or a lower alkyl group optionally substituted by one or more halogen atoms.

Of the compounds of formula (I) and their salts, preferred are heteroaryl-substituted acryloylbenzoguanidine derivatives and their salts where ring A is a 5-membered or 6-membered heteroaryl group which may optionally be substituted by one or more substituents selected from the members of the following group D and which may optionally be condensed with one or two benzene rings, and ring B is an aryl group which may optionally be substituted by one or more substituents selected from the members of the following group E.

### Group D, Group E:

A lower alkyl group optionally substituted by one or more halogen atoms, a lower alkenyl group, a lower alkynyl group, a cycloalkyl group, a lower alkoxy group, a lower alkoxy-lower alkoxy group, a lower alkoxycarbonyl-lower alkoxy group, a carboxyl-lower alkoxy group, an amino-lower alkoxy group, a mono- or di-lower alkylamino-lower alkoxy group, a nitrogen-containing, saturated heterocyclic group-substituted lower alkoxy group, a lower alkoxycarbonyl group, a carboxyl group, a halogen atom, a nitro group, a cyano group, an amino group, a mono- or di-lower alkylamino group, a lower alkanoyl group, a lower alkanoylamino group, a lower alkanoyl-lower alkylamino group, a lower alkanoyloxy group, a hydroxyl group, a mercapto group, a lower alkylthio group, a lower alkylsulfinyl group, a lower alkylsulfonyl group, an aminosulfonyl group, a mono- or di-lower alkylaminosulfonyl group, a lower alkylsulfonylamino group, a lower alkylsulfonylmono-lower alkylamino group, a carbamoyl group, a mono- or di-lower alkylaminocarbonyl group, a methylenedioxy group, an ethylenedioxy group, a propylenedioxy group, an optionally-substituted aryl group, an optionally-substituted aralkyl group, an optionally-substituted, 5-membered or 6-membered heteroaryl group, an optionally-substituted aryloxy group, and an optionally-substituted aralkyloxy group.

More preferred are heteroaryl-substituted acryloylguanidine derivatives of formula (I) and their salts where group D consists of a lower alkyl group optionally substituted by one or more halogen atoms, a lower alkenyl group, a lower alkynyl group, a lower alkoxy group, a halogen atom, a nitro group, a cyano group, an amino group, a mono- or di-lower alkylamino group, a lower alkanoyl group, a lower alkanoylamino group, a lower alkanoyl-lower alkylamino group, a lower alkanoyloxy group, a hydroxyl group, a mercapto group, a lower alkylthio group, a lower alkylsulfinyl group, a lower alkylsulfonyl group, an aminosulfonyl group, a mono- or di-lower alkylaminosulfonyl group, a lower alkylsulfonylamino group, a lower alkylsulfonyl-mono-lower alkylamino group, a carbamoyl group, and a mono- or di-lower alkylaminocarbonyl group, and group E consists of a lower alkyl group optionally substituted by one or more halogen atoms, a lower alkenyl group, a lower alkynyl group, a cycloalkyl group, a lower alkoxy group, a lower alkoxy-lower alkoxy group, a lower alkoxycarbonyl-lower alkoxy group, a carboxy-lower alkoxy group, an amino-lower alkoxy group, a mono- or di-lower alkylamino-lower alkoxy group, a nitrogen-containing, saturated heterocyclic group-substituted lower alkoxy group, a lower alkoxycarbonyl group, a carboxyl group, a halogen atom, a nitro group, a cyano group, an amino group, a mono- or di-lower alkylamino group, a lower alkanoyl group, a lower alkanoylamino group, a lower alkanoyl-lower alkylamino group, a lower alkanoyloxy group, a hydroxyl group, a mercapto group, a lower alkylthio group, a lower alkylsulfinyl group, a lower alkylsulfonyl group, an aminosulfonyl group, a mono- or di-lower alkylaminosulfonyl group, a lower alkylsulfonylamino group, a lower alkylsulfonyl-mono-lower alkylamino groups, a carbamoyl group, a mono- or di-lower alkylaminocarbonyl group, a methylenedioxy group, an ethylenedioxy group, a propylenedioxy group, an aryl group, an aralkyl group, a 5-membered or 6-membered heteroaryl group, an aryloxy group, and an aralkyloxy group.

Even more preferred are heteroaryl-substituted acryloylguanidine derivatives of formula (I) and their salts where R₁ is a hydrogen atom;
heteroaryl-substituted acryloylguanidine derivatives of formula (I) and their salts where ring A is a 5-membered or 6-membered heteroaryl group which may optionally be substituted by one or more substituents selected from a lower alkyl group and a lower alkanoylamino group and which may optionally be condensed with a benzene rings; and ring B is a phenyl group which may optionally be substituted by one or more substituents selected from a lower alkyl group optionally substituted by one or more halogen atoms, a lower alkoxy group, a lower alkoxycarbonyl-lower alkoxy group, a nitrogen-containing, saturated heterocyclic group-substituted lower alkoxy group, a halogen atom, a nitro group, a cyano group, an amino group, a hydroxyl group, a lower alkylsulfonyl group, an aryl group, an aryloxy group and an aralkyloxy group, or is an unsubstituted naphthyl group;
heteroaryl-substituted acryloylguanidine derivatives of formula (I) and their salts where ring A is a 5-membered or 6-membered heteroaryl group selected from a pyridyl group, a thienyl group, a thiazolyl group, a pyrrolyl group, a benzimidazolyl group and a pyrazinyl group; and
heteroaryl-substituted acryloylguanidine derivatives of formula (I) and their salts where ring A and ring B are cis-configured.

The present invention also relates to a pharmaceutical composition comprising a heteroaryl-substituted acryloylguanidine derivative of formula (I) or its salt, and a pharmaceutically-acceptable carrier, especially to that for the Na⁺/H⁺ exchange inhibitors. In particular, it relates to a pharmaceutical composition comprising such ingredients, which is usable in prevention, treatment and diagnosis of disorders to be caused by the promotion of the activities of the Na⁺/H⁺ exchange, such as hypertension, arrhythmia, angina pectoris, cardiac hypertrophy, organopathy to be induced by ischemic re-perfusion, disorders to be induced by the depression of blood flow in organo-transplantation, myocardial infarction, recurrence of myocardial infarction, ischemic organopathy, cell-proliferative disorders, arteriosclerosis, diabetic complications, cancer, vascular hypertrophy, tylosis and hypertrophy of tissues and organs, cardiofibrosis, pulmofibrosis, hepatofibrosis, renofibrosis, renal glomerulosclerosis, for protection of transplants, edema, cerebredema, chronic cardiopathy, cardiomyopathy, pulmonary embolism, acute and chronic nephropathy, cerebral infarction, chronic cerebrocirculation disorders, cranial nerves disorders, disorders caused by hyperglycemia, disorders as associated with insulin resistance, shocks, inflammatory disorders, pneumonopathy, bronchopathy, osteoporosis, and acid-base balance disorders.

Compounds of formula (I) of the present invention are described in detail hereinunder.

Unless otherwise specifically indicated, the terminology "lower" as referred to herein for the definitions of chemical formulae is directed to a linear or branched hydrocarbon chain having from 1 to 6 carbon atoms.

Therefore, the "lower alkyl group" as referred to herein includes, for example, a methyl group, an ethyl group, a propyl group, an isopropyl group, a butyl group, an isobutyl group, a sec-butyl groups, a tert-butyl group, a pentyl group, an isopentyl group, a neopentyl group, a tert-pentyl group, a 1-methylbutyl group, a 2-methylbutyl group, a 1,2-dimethylpropyl group, a hexyl group, an isohexyl group, a 1-methylpentyl group, a 2-methylpentyl group, a 3-methylpentyl group, a 1,1-dimethylbutyl group, a 1,2-dimethylbutyl group, a 2,2-dimethylbutyl group, a 1,3-dimethylbutyl group, a 2,3-dimethylbutyl group, a 3,3-dimethylbutyl group, a 1-ethylbutyl group, a 2-ethylbutyl group, a 1,1,2-trimethylpropyl group, a 1,2,2-trimethylpropyl group, a 1-ethyl-1-methylpropyl group, and a 1,-ethyl-2-methylpropyl group. Preferably, it is an alkyl group having from 1 to 3 carbon atoms, more preferably a methyl group or an ethyl group.

The "5-membered or 6-membered heteroaryl group" as referred to herein is a 5-membered or 6-membered heteroaryl group having from 1 to 4 hetero atoms selected from nitrogen, sulfur and oxygen atoms and include, for example, a furyl group, a thienyl group, a pyrrolyl group, an imidazolyl group, a pyrazolyl group, a thiazolyl group, an isothiazolyl group, an oxazolyl group, an isoxazolyl group, a triazolyl group, an oxadiazolyl group, a thiadiazolyl group, a tetrazolyl group, a pyridyl group, a pyrimidyl group, a pyridazinyl group and a pyrazinyl group.

The "5-membered or 6-membered heteroaryl group" of the "5-membered or 6-membered heteroaryl group which may optionally be substituted and may optionally be condensed with one or two benzene rings" for ring A may be any of the above-mentioned, 5-membered or 6-membered heteroaryl groups, and it may optionally be condensed with one or two benzene rings. The condensed ring includes, for example, an indolyl group, an indazolyl group, a benzofuranyl group, an isobenzofuranyl group, a benzothienyl group, an isoindolyl group, an isoquinolyl group, a chromenyl group, a quinolyl group, an quinazolinyl group, a benzimidazolyl group, a benzothiazolyl group, a benzoxazolyl group, a benzotriazolyl group, a benzoxadiazolyl group, a phthalazinyl group, a quinoxalinyl group, a cinnolinyl group, an acridinyl group, and a carbazolyl group. The bond of the condensed ring to the 2-position of the acryloyl group of formula (I) may extend from any of the carbon and nitrogen atoms on the heteroaryl group or from any carbon atom on the benzene ring(s).

The "5-membered or 6-membered heteroaryl group optionally condensed with one or two benzene rings" for ring A is preferably a furyl group, a thienyl group, a pyrrolyl group, an imidazolyl group, a pyrazolyl group, a thiazolyl group, a pyridyl group, a pyrimidinyl group, a pyridazinyl group, a pyrazinyl group, an indolyl group, a benzofuranyl group, a benzothienyl group, a quinolyl group, or a benzimidazolyl group, but is more preferably a thienyl group, a pyrrolyl group, a thiazolyl group, a pyridyl group, a pyrazinyl group, or a benzimidazolyl group.

The "5-membered or 6-membered heteroaryl group optionally condensed with one or two benzene rings" can be substituted at any desired positions. The substituents for the group may be any substituents which are generally used as substituents for heteroaryl groups, but are preferably those of group D mentioned hereinabove. More preferably, the substituents are selected from a lower alkyl group optionally substituted by one or more halogen atoms, a lower alkenyl group, a lower alkynyl group, a lower alkoxy group, a halogen atom, a nitro group, a cyano group, an amino group, a mono- or di-lower alkylamino group, a lower alkanoyl group, a lower alkanoylamino group, a lower alkanoyl-lower alkylamino group, a lower alkanoyloxy group, a hydroxyl group, a mercapto group, a lower alkylthio group, a lower alkylsulfinyl group, a lower alkylsulfonyl group, an aminosulfonyl group, a mono- or di-lower alkylaminosulfonyl group, a lower alkylsulfonylamino group, a lower alkylsulfonyl-mono-lower alkylamino group, a carbamoyl group, and a mono- or di-lower alkylaminocarbonyl group. Especially preferably, they are a lower alkyl group and a lower alkanoylamino group.

The "aryl group" as referred to herein means an aromatic hydrocarbon ring residue and is preferably an aryl group having from 6 to 14 carbon atoms. Concretely, it includes a phenyl group, a naphthyl group, an indenyl group, an anthryl group, and a phenanthryl group. Preferred are a phenyl group and a naphthyl group.

The "aryl group" of the "optionally-substituted aryl group" for ring B as referred to herein may be any of the above-mentioned aryl groups, which may optionally be substituted by one or more desired substituents. The substituents for the group may be any ordinary substituents that are generally used as substituents for aryl groups. Preferred are the substituents of group E as referred to hereinabove. More preferably, the substituents are selected from a lower alkyl group optionally substituted by one or more halogen atoms, a lower alkenyl group, a lower alkynyl group, a cycloalkyl group, a lower alkoxy group, a lower alkoxy-lower alkoxy group, a lower alkoxycarbonyl-lower alkoxy group, a carboxyl-lower alkoxy group, an amino-lower alkoxy group, a mono- or di-lower alkylamino-lower alkoxy group, a nitrogen-containing, saturated hetero ring-substituted lower alkoxy group, a lower alkoxycarbonyl group, a carboxyl group, a halogen atom, a nitro group, an cyano group, an amino group, a mono- or di-lower alkylamino group, a lower alkanoyl group, a lower alkanoylamino group, a lower alkanoyl-lower alkylamino group, a lower alkanoyloxy group, a hydroxyl group, a mercapto group, a lower alkylthio group, a lower alkylsulfinyl group, a lower alkylsulfonyl group, an aminosulfonyl group, a mono- or di-lower alkylaminosulfonyl group, a lower alkylsulfonylamino group, a lower alkylsulfonyl-mono-lower alkylamino group, a carbamoyl group, a mono- or di-lower alkylaminocarbonyl group, a methylenedioxy group, an ethylenedioxy group, a propylenedioxy group, an aryl group, an aralkyl group, a 5-membered or 6-membered heteroaryl group, an aryloxy group, and an aralkyloxy group. Especially preferably, the substituents are selected from a lower alkyl group optionally substituted by one or more halogen atoms, a lower alkoxy group, a lower alkoxycarbonyl-lower alkoxy group, a nitrogen-containing, saturated hetero ring-substituted lower alkoxy group, a halogen atom, a nitro group, a cyano group, an amino group, a hydroxyl group, a lower alkylsulfonyl group, an aryl group, an aryloxy group, and an aralkyloxy group.

The "halogen atom" includes fluorine, chlorine, bromine and iodine atoms.

The "lower alkyl group optionally substituted by one or more halogen atoms" includes, for example, a trifluoromethyl group, a chloromethyl group, a dichloromethyl group, a chlorofluoromethyl group, a chlorofluorobromomethyl group, a bromomethyl group, an iodomethyl group, a 1-fluoro-2-bromoethyl group, and a 1,2-dichloroethyl group. Preferred is a trifluoromethyl group.

The "lower alkenyl group" is a linear or branched alkenyl group having from 2 to 6 carbon atoms and includes, for example, a vinyl group, an allyl group, a 1-propenyl group, an isopropenyl group, a 1-butenyl group, a 2-butenyl group, a 3-butenyl group, a 2-methyl-1-propenyl group, a 2-methylallyl group, a 1-methyl-1-propenyl group, a 1-methylallyl group, a 1,1-dimethylvinyl group, a 1-pentenyl group, a 2-pentenyl group, a 3-pentenyl group, a 4-pentenyl group, a 3-methyl-1-butenyl group, a 3-methyl-2-butenyl group, a 3-methyl-3-butenyl group, a 2-methyl-1-butenyl group, a 2-methyl-2-butenyl group, a 2-methyl-3-butenyl group, a 1-methyl-1-butenyl group, a 1-methyl-2-butenyl group, a 1-hexenyl group, and a 1-methyl-1-pentenyl group.

The "lower alkynyl group" is a linear or branched alkynyl group having from 2 to 6 carbon atoms and includes, for example, an ethynyl group, a 1-propynyl group, a 2-propynyl group, a 1-butynyl group, a 2-butynyl group, a 3-butynyl group, a 1-methyl-2-propynyl group, a 1-pentynyl group, a 2-pentynyl group, a 3-pentynyl group, a 4-pentynyl group, a 3-methyl-1-butynyl group, a 2-methyl-3-butynyl group, a 1-methyl-2-butynyl group, a 1-methyl-3-butynyl group, a 1,1-dimethyl-2-propynyl group, a 1-hexynyl group, a 2-hexynyl group, a 3-hexynyl group, a 4-hexynyl group, and a 5-hexynyl group.

The "cycloalkyl group" is preferably a cycloalkyl group having from 3 to 8 carbon atoms and includes, for example, a cyclopropyl group, a cyclobutyl group, a cyclopentyl group, a cyclohexyl group, a cycloheptyl group, and a cyclooctyl group.

The "lower alkoxy group" means a group to be derived from a hydroxyl group by substituting its hydrogen atom with any of the above-mentioned lower alkyl groups, and this includes, for example, a methoxy group, an ethoxy group, a propoxy group, an isopropoxy group, a butoxy group, an isobutoxy group, a sec-butoxy group, a tert-butoxy group, a pentyloxy (amyloxy) group, an isopentyloxy group, a tert-pentyloxy group, a neopentyloxy group, a 2-methylbutoxy group, a 1,2-dimethylpropoxy group, a 1-ethylpropoxy group, and a hexyloxy group. Preferably, it is a methoxy group, an ethoxy group or a propoxy group.

The "lower alkoxy-lower alkoxy group", the "lower alkoxycarbonyl-lower alkoxy group", the "carboxyl-lower alkoxy group", the "amino-lower alkoxy group", the "monoor di-lower alkylamino-lower alkoxy group" and the "nitrogen-containing, saturated heterocyclic group-substituted lower alkoxy group" are substituted lower alkoxy groups which are derived from any of the above-mentioned alkoxy groups by substituting its one hydrogen atom with any of a lower alkoxy group, a lower alkoxycarbonyl group, a carboxyl group, an amino group, a mono- or di-lower alkylamino group, and a nitrogen-containing, saturated heterocyclic group, respectively. Concretely, the "lower alkoxy-lower alkoxy group" includes, for example, a methoxymethoxy group, an ethoxymethoxy group, a propoxymethoxy group, an isopropoxymethoxy group, a butoxymethoxy group, an isobutoxymethoxy group, a pentyloxymethoxy group, a hexyloxymethoxy group, a methoxyethoxy group, an ethoxyethoxy group, and a propoxyethoxy group.

The "nitrogen-containing, saturated heterocyclic group" is a 5-membered to 8-membered, nitrogen-containing, saturated heterocyclic group having a bond on its nitrogen atom, which may optionally have any one of an oxygen atom, a sulfur atom and a group of N-R₂ (where R₂ represents a hydrogen atom or a lower alkyl group). Concretely, it includes, for example, a pyrrolidinyl group, a piperidyl group, a morpholinyl group, a piperazinyl group, a pyrazolidinyl group, an imidazolidinyl group, and a homopiperazinyl group. These piperazinyl group, pyrazolidinyl group, imidazolidinyl group and homopiperazinyl group may optionally be substituted by a lower alkyl group on their nitrogen atom. Preferred is a piperidinyl group.

The "lower alkoxycarbonyl group" is a carbonyl group as substituted by any of the above-mentioned lower alkoxy groups, and this includes, for example, a methoxycarbonyl group, an ethoxycarbonyl group, a propoxycarbonyl group, an isopropoxycarbonyl group, a butoxycarbonyl group; an isobutoxycarbonyl group, a pentyloxycarbonyl group, and a hexyloxycarbonyl group.

The "mono- or di-lower alkylamino group" is an amino group, of which one or two hydrogen atoms are substituted by any of the above-mentioned alkyl groups. In the di-lower alkylamino group, the two alkyl groups may be the same or different. The mono-lower alkylamino group includes, for example, a methylamino group, an ethylamino group, a propylamino group, an isopropylamino group, a butylamino group, an isobutylamino group, a sec-butylamino group, a tert-butylamino group, and a pentylamino group. The di-lower alkylamino group includes, for example, a dimethylamino group, a diethylamino group, a dipropylamino group, a methylethylamino group, a methylpropylamino group, a methylisopropylamino group, a methylbutylamino group, a methylisobutylamino group, an ethylpropylamino group, and an ethylisopropylamino group.

The "lower alkanoyl group" includes, for example, a formyl group, an acetyl group, a propionyl group. a butyryl group, an isobutyryl group, a valeryl group, an isovaleryl group, a pivaloyl group, and a hexanoyl group.

The "lower alkanoylamino group" includes, for example, a formylamino group, an acetylamino group, a propionylamino group. a butyrylamino group, an isobutyrylamino group, a valerylamino group, an isovalerylamino group, a pivaloylamino group, and a hexanoylamino group.

The "lower alkanoyl-lower alkylamino group" is a group to be derived from any of the above-mentioned lower alkanoylamino group by substituting another hydrogen atom of its amino group with any of the above-mentioned alkyl groups. Concretely, this includes, for example, an N-acetyl-N-methylamino group, and an N-acetyl-N-ethylamino group.

The "lower alkanoyloxy group" includes, for example, a formyloxy group, an acetoxy group, a propionyloxy group. a butyryloxy group, an isobutyryloxy group, a valeryloxy group, an isovaleryloxy group, a pivaloyloxy group, and a hexanoyloxy group.

The "lower alkylthio group", the "lower alkylsulfinyl group" and the "lower alkylsulfonyl group" are groups to be derived from a mercapto group, a sulfinyl group and a sulfonyl group, respectively, by substituting its hydrogen atom with any of the above-mentioned lower alkyl groups. Concretely, the lower alkylthio group includes, for example, a methylthio group, an ethylthio group, a propylthio group, an isopropylthio group, a butylthio group, an isobutylthio group, a sec-butylthio group, a tert-butylthio group, a pentylthio group, an isopentylthio group, a neopentylthio group, a tert-pentylthio group, a 1-methylbutylthio group, a 2-methylbutylthio group, and a 1,2-dimethylpropylthio group.

The "mono- or di-lower alkylaminosulfonyl group" means a group as derived from an aminosulfonyl group by substituting its one or two hydrogen atoms with a lower alkyl group, which is represented by a general formula - SO₂-NRR' (where R represents a lower alkyl group, and R' represents a hydrogen atom or a lower alkyl group). In the di-lower alkylaminosulfonyl groups, the two alkyl groups may be the same or different. The mono-lower alkylaminosulfonyl group includes, for example, an N-methylaminosulfonyl group, an N-ethylaminosulfonyl group, an N-propylaminosulfonyl group, an N-isopropylaminosulfonyl group, and an N-butylaminosulfonyl group. The di-lower alkylaminosulfonyl group includes, for example, an N,N-dimethylaminosulfonyl group, an N,N-diethylaminosulfonyl group, an N,N-dipropylaminosulfonyl group, an N-methyl-N-ethylaminosulfonyl group, an N-methyl-N-propylaminosulfonyl group, an N-ethyl-N-isopropylaminosulfonyl group, an N-methyl-N-butylaminosulfonyl group, an N-ethyl-N-propylaminosulfonyl group, and an N-ethyl-N-isopropylaminosulfonyl group.

The "lower alkylsulfonylamino group" means a group as derived from an amino group by substituting its one hydrogen atom with a lower alkylsulfonyl group, and this is represented by a general formula -NH-SO₂-R (where R represents a lower alkyl group). Concretely, this includes, for example, a methylsulfonylamino group, an ethylsulfonylamino group, a propylsulfonylamino group, an isopropylsulfonylamino group, a butylsulfonylamino group, an isobutylsulfonylamino group, and a pentylsulfonylamino group.

The "lower alkylsulfonyl-mono-lower alkylamino group" means a group as derived from an amino group by substituting its two hydrogen atoms with a lower alkylsulfonyl group and a lower alkyl group, and this is represented by a general formula -NR-SO₂-R (where R and R' may be the same or different and each represents a lower alkyl group). Concretely, this includes, for example, an N-methylsulfonyl-N-methylamino group, an N-ethylsulfonyl-N-methylamino group, an N-propylsulfonyl-N-methylamino group, an N-methylsulfonyl-N-ethylamino group, an N-ethylsulfonyl-N-ethylamino group, an N-propylsulfonyl-N-ethylamino group, an N-methylsulfonyl-N-propylamino group, and an N-methylsulfonyl-N-isopropylamino group.

The "mono- or di-lower alkylaminocarbonyl group" is a group to be represented by a general formula -C(=O)NRR' (where R represents a lower alkyl group, and R' represents a hydrogen atom or a lower alkyl group). In the di-lower alkylaminocarbonyl group, the two alkyl groups may be the same or different. Concretely, the mono-lower alkylaminocarbonyl group includes, for example, an N-methylaminocarbonyl group, an N-ethylaminocarbonyl group, an N-propylaminocarbonyl group, an N-isopropylaminocarbonyl group, an N-butylaminocarbonyl group, and an N-isobutylaminocarbonyl group; and the di-lower alkylaminocarbonyl group includes, for example, an N,N-dimethylaminocarbonyl group, an N,N-diethylaminocarbonyl group, and an N-methyl-N-ethylaminocarbonyl group.

The "aralkyl group" means a group as derived from any of the above-mentioned lower alkyl groups by substituting its one or more hydrogen atoms with any of the above-mentioned aryl groups. Referring to a phenyl group as its aryl group moiety, the aralkyl group includes, for example, a benzyl group, a phenethyl group, a 1-phenylethyl group, a 3-phenylpropyl group, a 1-phenylpropyl group, and a 1-methyl-2-phenylpropyl group.

The "aryloxy group" and the "aralkyloxy group" are groups as derived from a hydroxyl group by substituting its hydrogen atom with any of the above-mentioned aryl groups and aralkyl groups, respectively. Concretely, the aryloxy group includes, for example, a phenoxy group, and a naphthyloxy group; and the aralkyloxy group includes, for example, a benzyloxy group, a phenethyloxy group, and a 1-phenylethoxy group.

The substituents for the "optionally-substituted aryl group", the "optionally-substituted aralkyl group", the "optionally-substituted, 5-membered or 6-membered heteroaryl group", the "optionally-substituted aryloxy group", and the "optionally-substituted aralkyl group" in group D and group E are not specifically defined but may be any ordinary ones that are generally employed for the groups. Concretely, they include, for example, a lower alkyl group optionally substituted by one or more halogen atoms, a lower alkoxy group, a hydroxyl group, a halogen atom, a carboxyl group, an alkoxycarbonyl group, a nitro group, a cyano group, an amino group, a mono- or di-lower alkylamino group, a lower alkanoyl group, and a lower alkanoylamino group.

The compounds of the present invention include cis/trans (or (E)/(Z)) geometrical isomers, depending on the double bond of the acryloyl group therein. The present invention encompasses any of separated, (E) or (Z) isomers as well as their mixtures. Of the compounds of the present invention, especially preferred are those where ring A and ring B are cis-configured.

In addition, the compounds of the present invention include tautomers, depending on the guanidino carbonyl group therein. The present invention also encompasses any of separated tautomers and their mixtures.

Also depending on the substituents in them, the compounds of the present invention may include other various geometrical isomers and tautomers. Naturally, the present invention encompasses any of separated, such isomers and tautomers as well as their mixtures.

The compounds of the present invention may have an asymmetric carbon atom, and may include (R)/(S) optical isomers, depending on the asymmetric carbon atom, if any. The present invention encompasses any of isolated, such optical isomers and also their mixtures.

The compounds of the present invention may be in the form of their acid-added salts. The salts are not specifically defined, provided that they are pharmaceutically acceptable. Concretely, the salts include, for example, acid-added salts with any of inorganic acids, such as hydrochloric acid, hydrobromic acid, hydroiodic acid, sulfuric acid, nitric acid and phosphoric acid, or with any of organic acids, such as formic acid, acetic acid, propionic acid, oxalic acid, malonic acid, succinic acid, fumaric acid, maleic acid, lactic acid, malic acid, tartaric acid, citric acid, methanesulfonic acid, ethanesulfonic acid, aspartic acid and glutamic acid. Especially preferred are salts with methanesulfonic acid.

In addition, the present invention also encompasses various hydrates and solvates of the compounds of formula (I) and their salts, as well as their polymorphic crystals.

### (Production Method)

The compounds and their salts of the present invention can be produced in accordance with various known methods, while utilizing their characteristics based on their basic skeletons and substituents. One typical example of producing them is described below. 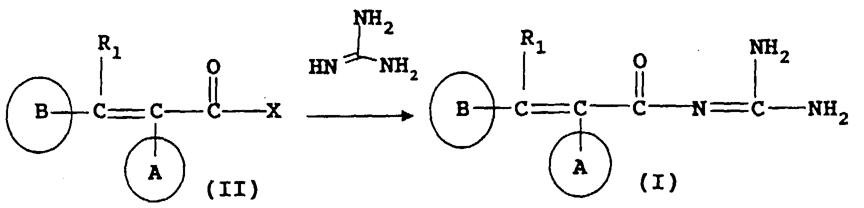 wherein ring A, ring B and R₁ have the same meanings as those mentioned hereinabove; and X represents a hydroxyl group or a leaving group that can be easily substituted with a nucleophilic reagent.

As in the above, the compounds (I) of the present invention can be produced by reacting a 3-aryl-2-heteroaryl-acrylic acid or its reactive derivative of formula (II) with guanidine.

The leaving group X that can be easily substituted with a nucleophilic reagent includes, for example, a halogen atom, a lower alkoxy group and an aralkyloxy group.

The reactive derivatlves of carboxylic acids having such a leaving group include, for example, acid halides, acid anhydrides, activated esters, lower alkyl esters, and acid azides.

Concretely, for example, the acid halides include acid chlorides and acid bromides.

The acid anhydrides include, for example, symmetric acid anhydrides and mixed acid anhydrides. Specific examples of mixed acid anhydrides include mixed acid anhydrides with alkyl chlorocarbonates such as ethyl chlorocarbonate and isobutyl chlorocarbonate; mixed acid anhydrides with aralkyl chlorocarbonates such as benzyl chlorocarbonate; mixed acid anhydrides with aryl chlorocarbonates such as phenyl chlorocarbonate; and mixed acid anhydrides with alkanoic acids such as isovaleric acid and pivalic acid.

The activated esters include, for example, p-nitrophenyl esters, N-hydroxysuccinimide esters, pentafluorophenyl esters, 2,4,5-trichlorophenyl esters, pentachlorophenyl esters, cyanomethyl esters, N-hydroxyphthalimide esters, N-hydroxy-5-norbornene-2,3-dicarboxyimide esters, N-hydroxypiperidine esters, 8-hydroxyquinoline esters, 2-hydroxyphenyl esters, 2-hydroxy-4,5-dichlorophenyl esters, 2-hydroxypyridine esters, 2-pyridylthiol esters, and 1-benzotriazolyl esters.

Such activated derivatives of carboxylic acids can be easily obtained from the corresponding carboxylic acids in accordance with ordinary known methods.

The reaction with acid halides or acid anhydrides can be conducted in the presence of a base or of an excess amount of guanidine in a solvent, while cooling or at room temperature.

The base includes, for example, inorganic bases such as sodium hydroxide, potassium hydroxide, sodium carbonate, potassium carbonate and sodium hydrogencarbonate, as well as organic bases such as triethylamine, tributylamine, diisopropylethylamine, N-methylmorpholine, pyridine and 4-dimethylaminopyridine.

The solvent includes, for example, aromatic hydrocarbon solvents such as benzene, toluene and xylene; ether solvents such as tetrahydrofuran and 1,4-dioxane; halogenated hydrocarbon solvents such as dichloromethane, chloroform and 1,2-dichloroethane; amide solvents such as dimethylformamide and dimethylacetamide: and basic solvents such as pyridine. These solvents can be used either singly or as combined. The solvent shall be suitably selected, depending on the starting compounds to be reacted therein.

The reaction with ester derivatives can be conducted in the presence of an equimolar or excess amount of guanidine in a solvent, while cooling or heating or at room temperature. As the case may be, the reaction system can be heated at about 130°C for a short period of time, after the solvent used has been removed therefrom through distillation.

The solvent includes, for example, ether solvents such as tetrahydrofuran, 1,2-dimethoxyethane and 1,4-dioxane; and amide solvents such as dimethylformamide and dimethylacetamide. These solvents can be used either singly or as combined. For the reaction with other esters, for example, employable are alcohol solvents such as methanol, ethanol and isopropanol; ether solvents such as tetrahydrofuran, 1,2-dimethoxyethane and 1,4-dioxane; and amide solvents such as dimethylformamide and dimethylacetamide. These solvents can also be used either singly or as combined.

Where X is a hydroxyl group, it is desirable that the reaction is conducted in the presence of a dehydrating agent. The dehydrating agent includes, for example, dicyclohexylcarbodiimide (DCC), diisopropylcarbodiimide (DIPC), 1-ethyl-3-(3-dimethylaminopropyl)carbodiimide (WSC), benzotriazol-1-yl-tris(dimethylamino)phosphonium hexafluorophosphide (BOP), diphenylphosphonylazide (DPPA), and 1,1'-carbonyldi-1H-imidazole (CDI). As the case may be, additives such as N-hydroxysuccinimide (HONSu), 1-hydroxybenzotriasol (HOBt), and 3-hydroxy-4-oxo-3,4-dihydro-1,2,3-benzotriazine (HOObt) can be added to the reaction system.

The solvent includes, for example, aromatic hydrocarbon solvents such as benzene, toluene and xylene; ether solvents such as tetrahydrofuran and 1,4-dioxane; halogenated hydrocarbon solvents such as dichloromethane, chloroform and 1,2-dichloroethane; amide solvents such as dimethylformamide and dimethylacetamide; and basic solvents such as pyridine. These solvents can be used either singly or as combined.

Where 3-aryl-2-heteroaryl-acrylic acid derivatives (II) have a functional group that is sensitive to the reaction, such as a hydroxyl group or an amino group, the functional group is previously protected with a protecting group in accordance with known methods, then the thus-protected compound is reacted with guanidine in the manner as mentioned hereinabove, and thereafter the protecting group is removed from the product to obtain the intended compound (I) of the present invention.

### (Methods for Producing Starting Compounds)

The 3-aryl-2-heteroaryl-acrylic acids and their activated derivatives (II), which are the starting compounds in the method of producing the compounds (I) of the invention, can be produced in accordance with various known methods. For example, they can be produced according to the methods mentioned below. 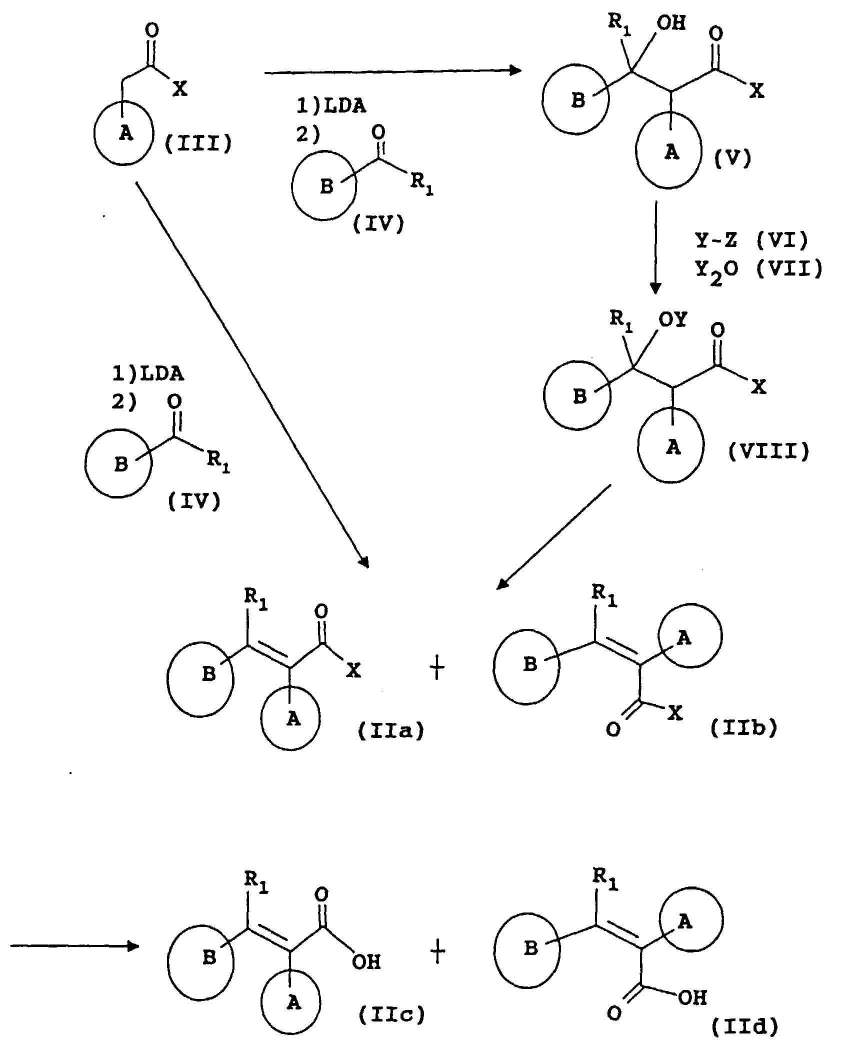 wherein ring A, ring B, R₁ and X have the same meanings as those mentioned hereinabove; Y represents a lower alkanoyl group optionally substituted by one or more halogen atoms, a mesyl group, a tosyl group, or a trifluoromethanesulfonyl group; and Y represents a halogen atom.

As in the above, a heteroaryl-acetate derivative of formula (III) is reacted with lithium diisopropylamide (LDA) in an inert solvent such as tetrahydrofuran, while cooling, preferably at from -80 to -60°C, and then coupled with a carbonyl derivative of formula (IV) in an inert solvent. Varying the conditions for the reaction, the coupling reaction may often produce directly a single compound of 3-aryl-2-heteroaryl-acrylic acid derivatives of formulae (IIa) and (IIb) or a mixture thereof, without isolating the intermediates. As the case may be, the coupling reaction may also produce a 3-aryl-2-heteroarylpropionate of formula (V). In this case, the ester derivative (V) is further reacted with an acid halide of formula (VI) or with an acid anhydride of formula (VII) in the presence of a base to thereby esterify the hydroxyl group of the ester derivative (V) to give a compound of formula (VIII). Next, the compound (VIII) is treated with a base at room temperature or under heat to remove the acid moiety (YOH) therefrom, whereby a single compound or mixture of 3-aryl-2-heteroaryl-acrylic acid derivatives of formulae (IIa) and (IIb) can be obtained.

The acid halide includes, for example, acetyl chloride, trifluoroacetyl chloride, methanesulfonyl chloride, tosyl chloride and trifluoromethanesulfonyl chloride; and the acid anhydride includes, for example, acetic anhydride and trifluoroacetic anhydride. In the above-mentioned process, it is also possible to directly obtain a single compound or mixture of acid derivatives of formulae (IIa) and (IIb), without isolating the ester compound of formula (VIII).

Where the 3-aryl-2-heteroaryl-acrylic acid derivatives of formulae (IIa) and (IIb) are esters, they are hydrolyzed to give 3-aryl-2-heteroaryl-acrylic acids of formulae (IIc) and (IId). 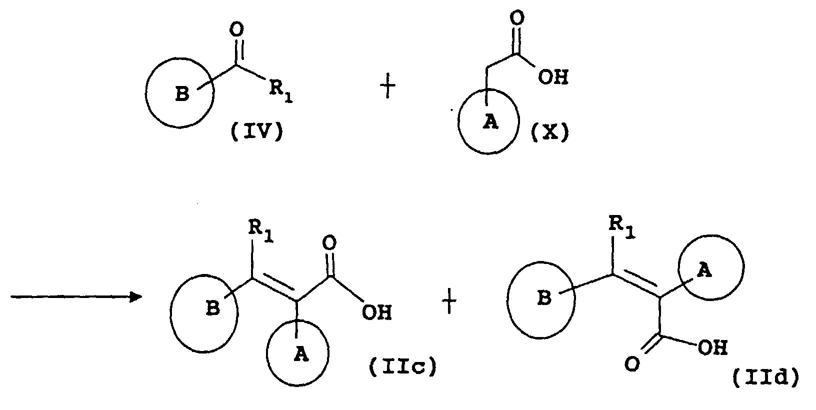 wherein ring A, ring B and R₁ have the same meanings as mentioned hereinabove.

As in the above, a carbonyl derivative (IV) and a heteroaryl-acetic acid derivative (X) can be conducted at room temperature or heating in acetic anhydride in the presence of a base such as triethylamine, to produce the starting compounds (IIc) and (IId) (see references, Org. Synth. Coll., Vol. IV, 730-731 (1963); Org. Synth. Coll. Vol. IV, 777-779 (1963)).

The reaction products as obtained according to any of the above-mentioned methods are isolated and purified as their free compounds, their salts or various their solvates such as hydrates. The salts can be produced through ordinary salt formation. The isolation and purification of the products can be conducted through ordinary chemical means such as extraction, concentration, distillation, crystallization, filtration, recrystallization, and various chromatographic means.

Isomeric mixtures of the products can be isolated into the individual isomers by any ordinary methods utilizing the physico-chemical difference between them. For example, mixtures of optical isomers can be isolated through ordinary racemic resolution of, for example, partitioning crystallizaiton or chromatography. If desired, optical isomers can be produced, starting from suitable optically active compounds.

### INDUSTRIAL APPLICABILITY

The compounds of the present invention are useful as active ingredients in pharmaceutical compositions. In particular, since the compounds have the action of inhibiting the activity of cellular, sodium-proton exchange mechanism (Na⁺/H⁺ exchanger), they are useful in prevention, treatment and diagnosis of various disorders as associated with the physiological activities and the symptoms of diseases in which the Na⁺/H⁺ exchange participate.

Concretely, the compounds of the present invention are useful in prevention and treatment of various disorders to be caused by the promoted activity of the Na⁺/H⁺ exchange, for example, hypertension (e.g., essential hypertension, as well as secondary hypertension that shall follow the primary disorders such as renal insufficiency, primary aldosteronism, adrenal oxygen insufficiency, Liddle's syndrome, Gordon's syndrome, renal tubular acidosis (RTA) type IV, hyporeninosic hypoaldosteronism, double renovascular hypertension), arrhythmia, angina pectoris, cardiac hypertrophy, organopathy to be induced by ischemicreperfusion (e.g., disorders in cardiac ischemic reperfusion, disorders to be caused by surgical operations (such as organo-transplantation, PTCA)), disorders to be induced by the depression of blood flow in organo-transplantation, myocardial infarction and for prevention of recurrence of myocardial infarction (for example, for secondary prevention of myocardial infarction, and for prevention of cardiac accidents), for protection of ischemic organs and for removal of ischemic symptoms (of, for example, disorders that follow the ischemia in heart, brain, kidney, stomach, intestines, lung, liver and skeletal muscle, in particular, disorders to follow cerebral infarction, disorders of sequela to follow cerebral paralysis, and disorders to follow cerebral edema), cell-proliferative disorders, arteriosclerosis, diabetic complications (e.g., retinopathy, nephropathy), cancer, vascular hypertrophy (e.g., arteriosclerotic disorders, arteritis, re-hematostenosis after PCTA, hematostenosis in vascular transplantation), tylosis and hypertrophy of tissues and organs (e.g., tylosis and hypertrophy of heart, kidney, prostate, smooth muscle tissue, etc.), cardiofibrosis, pulmofibrosis, hepatofibrosis, renofibrosis, renal glomerulosclerosis, for protection of transplants, edema, cerebral edema, chronic cardiopathy (e.g., cardiac insufficiency), cardiomyopathy, pulmonary embolism, acute and chronic nephropathy (e.g., renal insufficiency), cerebral infarction, chronic cerebrocirculation disorders (e.g., cerebral paralysis), cranial nerves disorders (e.g., dementia, AIDS), disorders caused by hyperglycemia (e.g., diabetic neuropathy, hyperlipemia), disorders as associated with insulin resistance, shocks (e.g., allergic, cardiac, blood flow depression-induced and bacterial shocks), inflammatory disorders, pneumonopathy, bronchopathy, osteoporosis, and acid-base balance disorders.

In addition, the compounds of the present invention can be used in diagnosis of hypertension, diabetes and arteriosclerosis in which sodium-proton exchange mechanism (Na⁺/H⁺ exchanger) participate.

The effects of the compounds of the present invention have been verified by the following pharmacological tests.

### (1) Test for the activity of inhibiting the swelling of rabbit platelets:

### Principle

When being loaded with an acid, platelets excrete H⁺ ions out of their cells, thereby activating the Na⁺/H⁺ exchange existing therein. In this process, if Na⁺ ions exist in the external liquid around platelets, the activation of the Na⁺/H⁺ exchange shall result in the intake of Na⁺ ions into the cells. The resulting introduction of Na⁺ ions into the cells shall further result in the intake of water thereinto due to the osmotic pressure gradient, whereby the platelets are swollen.

### Preparation of platelet-rich plasma (PRP)

A rabbit was anesthetized with pentobarbital (30 mg/kg, as intravenous injection through auricle) and fixed at supination. Its skin at the cervical part was cut and the carotid artery was exposed out, through which the blood was collected into a plastic centrifuge tube containing therein 1/10 volume of an anticoagulant, ACD-A (acid-citrate dextrose, produced by Terumo Co.), while using a polyethylene cannula (intravenous catheter (5 Fr), produced by Atom Co.). Thus was obtained about 100 ml of blood from a rabbit of about 2 kg body weight. After having been well blended in the tube, the resulting mixture was centrifuged at room temperature, at 1400 rpm (350 x g) for 10 minutes. The supernatant thus obtained is the intended PRP. About 20 to 40 ml of PRP was obtained from 100 ml of the blood.

### Measurement of the activity of the Na⁺/H⁺ exchange through the swelling reaction of platelets

The degree of swelling of platelets was measured through the detection of the change in the absorbance of the PRP sample, using a spectrophotometer (U-3000 Model, produced by Hitachi Ltd.) equipped with a personal computer (Vectra 286/12 Model, produced by Yokokawa Hewlett-Packard Co.). Precisely, a plastic cuvet containing therein a propionate solution (970 µl), which is to apply acid loading to the PRP sample, was prepared. The PRP sample (140 µl) was added thereto, and immediately its absorbance was measured at 680 nm. The variation in the absorbance was represented by the exponential decrease, which reached the plateau within from 3 to 4 minutes. A chemical substance to be tested herein was dissolved in DMSO at a concentration of 10 mM, and then diluted with a propionate solution to the optimum concentration. The thus-diluted solution comprising the chemical substance along with a propionate was used herein. The swelling of the platelets in the PRP sample shall be inhibited, if the Na⁺ ion concentration in the external liquid around the platelets is decreased or if the concentration of the chemical substance (this has an action of inhibiting the activity of the Na⁺/H⁺ exchange) as applied to the PRP sample is increased. The propionate solution employed herein was comprised of the following components.
140 mM of sodium propionate; 20 mM of N-(2-hydroxyethyl)piperazine-N'-2-ethanesulfonic acid (HEPES); 10 mM of glucose; 5 mM of potassium chloride; 1 mM of magnesium chloride; 1 mM of calcium chloride; pH 6.7.

In order to qualitatively determine the Na⁺/H⁺ exchange-inhibiting activity of the test chemical, the variation in the absorbance per the unit time was calculated from the data of the variation in the absorbance obtained herein and plotted logarithmically in a graph. From the initial gradient of the graph, the rate constant for the swelling reaction was obtained. It has been confirmed that the rate constant does not depend on the number of platelets in samples.

Finally, the rate constants at varying concentrations of the test chemical were plotted in a Dixon graph (where the X axis indicates the concentration of the test chemical, and Y axis indicates the reciprocal of the rate constant), from which obtained was an inhibitory constant (Ki value) that is intrinsic to the test chemical.

### Test Results

The test results have verified that the compounds of the present invention have a Ki value of at least on 10⁻⁷ order and therefore have a high activity of inhibiting the Na⁺/H⁺ exchange.

### (2) In a test of inhibiting arrhythmia as induced by continuous injection of ouabain (combined with lactic acid loading) into test animals, the compounds of the present invention were verified to have a high activity of inhibiting arrhythmia.

The pharmaceutical composition of the present invention comprising any of compounds (I) and their salts along with a pharmaceutically-acceptable carrier can be prepared according to known methods by mixing one or more of the compounds (I) and their salts with any of pharmaceutical carriers, vehicles and other additives which are generally used in preparing ordinary pharmaceutical compositions. It can be administered either perorally in the form of, for example, tablets, pills, capsules, granules, powders or liquids, or parenterally in the form of, for example, intravenous or intramuscular injections, suppositories or endermic preparations.

The peroral solid composition of the present invention may be in any form of tablets, powders, granules, etc. It comprises one or more active ingredients as mixed with at least one inert diluent which may be selected from, for example, lactose, mannitol, glucose, hydroxypropyl cellulose, microcrystalline cellulose, starch, polyvinyl pyrrolidone and magnesium metasilicate aluminate. The composition may additionally comprise, like ordinary pharmaceutical preparations, any additives other than the inert diluent. The additives include, for example, a lubricant such as magnesium stearate, a disintegrator such as calcium cellulose glycolate, a stabilizer such as lactose, and a dissolution aid such as glutamic acid and aspartic acid. If desired, the tablets or pills of the pharmaceutical composition of the present invention may be sugar-coated ones or may be coated with film of a substance that is soluble in the stomach or intestines, such as sucrose, gelatin, hydroxypropyl cellulose or hydroxypropylmethyl cellulose phthalate.

The peroral liquid composition of the present invention may contain pharmaceutically-acceptable emulsifier, solvent, suspending agent, syrup, elixir and the like, and may contain an ordinary, inert diluent such as pure water or ethanol. In addition to such an inert diluent, the composition may also contain other auxiliary agents such as a wetting agent and a suspending agent, as well as a sweetener, a flavoring, an aromatic substance and a preservative.

The parenteral injection of the composition of the present invention may contain germ-free, aqueous or non-aqueous solvent, suspending agent and emulsifier. The aqueous solvent and suspending agent include, for example, distilled water for injection and physiological saline. the non-aqueous solvent and suspending agent include, for example, propylene glycol, polyethylene glycol, vegetable oils such as olive oil, alcohols such as ethanol, and Polysorbate 80. The injectable composition may further contain other auxiliary agents, such as a preservative, a wetting agent, an emulsifier, a dispersing agent, a stabilizer (e.g., lactose), a dissolution aid (e.g., glutamic acid, aspartic acid). The composition is sterilized by filtering it through a bacteria-trapping filter, or by adding thereto a microbicide, or by irradiating it. If desired, the composition may be prepared in the form of a solid, which can be dissolved in germ-free water or germ-free solvent for injection before use.

For peroral administration of the composition of the present invention, the dose of the active ingredient is suitably from about 0.001 to 10 mg per kg of the body weight a day, which may be administered once a day or may be divided into from 2 to 4 parts for separate administration a day. For intravenous administration of the composition of the present invention, the dose of the active ingredient is suitably from about 0.0001 to 1 mg per kg of the body weight a day, which may be administered once a day or may be divided into plural parts for separate administration a day. The dose shall be suitably determined, depending on the condition, the age and the sex of the patient.

### BEST MODES FOR CARRYING OUT THE INVENTION

The present invention is described in more detail hereinunder with reference to the following examples, which, however, are not intended to restrict the scope of the present invention. The following reference examples demonstrate the preparation of the starting compounds used in the examples.

### Reference Example 1:

14.5 ml of a solution of 1.69 M of n-butyl lithium in n-hexane was dropwise added to a solution of 2.460 g of diisopropylamine in 40 ml of tetrahydrofuran, at -50°C under argon atmosphere, and stirred for 20 minutes. A solution of 5.001 g of ethyl 2-thienylacetate in 3 ml of tetrahydrofuran was dropwise added thereto at -70°C, and stirred for 70 minutes, and thereafter a solution of 3.272 g of m-methoxybenzaldehyde in 5 ml of tetrahydrofuran was dropwise added thereto. Its temperature was elevated up to room temperature over a period of 2 hours, and this was then stirred at room temperature for 17 hours. 1N hydrochloric acid was added to the reaction mixture, whereby the aqueous layer of the mixture was made weakly acidic and then extracted with ethyl acetate. The resulting extract was dried with anhydrous magnesium sulfate, then the solvent was removed through distillation under reduced pressure, and the residue was purified 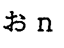 silica gel column chromatography (n-hexane/ethyl acetate) to give 1.724 g of ethyl (E)-3-(3-methoxyphenyl)-2-(2-thienyl)acrylate.

In the same manner as in Reference Example 1, compounds of the following Reference Examples 2 and 3 were obtained.

### Reference Example 2:

Ethyl (E)-3-(3-methoxyphenyl)-2-(3-thienyl)acrylate

### Reference Example 3:

Ethyl (E)-3-(2-chlorophenyl)-2-(3-pyridyl)acrylate

### Reference Example 4:

20 ml of an aqueous solution of 1N sodium hydroxide was added to a solution of 1.649 g of ethyl (E)-3-(3-methoxyphenyl)-2-(2-thienyl)acrylate in 20 ml of ethanol, and stirred at room temperature for 15 hours. The reaction mixture was concentrated to about a half, and its aqueous layer was made strongly acidic by adding thereto 1N hydrochloric acid and then extracted with chloroform. The resulting extract was dried with anhydrous magnesium sulfate, then the solvent was removed through distillation under reduced pressure, and the residue was washed with hexane and diethyl ether to give 1.017 g of (E)-3-(3-methoxyphenyl)-2-(2-thienyl)acrylic acid.

In the same manner as in Reference Example 4, compounds of the following Reference Examples 5 and 6 were obtained.

### Reference Example 5:

(E)-3-(3-methoxyphenyl)-2-(3-thienyl)acrylic acid

### Reference Example 6:

(E)-3-(2-chlorophenyl)-2-(3-pyridyl)acrylic acid

### Reference Example 7:

A mixture comprised of 2.873 g of 3-methoxybenzaldehyde, 3.001 g of 3-thiopheneacetic acid, 2 ml of triethylamine and 4 ml of acetic anhydride was stirred at 100°C for 3.5 hours and then concentrated. This was subjected to azeotropy with toluene and then purified on silica gel column chromatography (chloroform/methanol) to give 1.277 g of (E)-3-(3-methoxyphenyl)-2-(3-thienyl)acrylic acid.

### Reference Example 8:

A mixture comprised of 2 g of 3-pyridylacetic acid hydrochloride, 1.94 g of 2-chlorobenzaldehyde, 1.5 ml of triethylamine and 3 ml of acetic anhydride was stirred at 100°C for 2 hours and then concentrated. This was subjected to azeotropy with toluene, and the crystals thus precipitated out were recrystallized from ethanol to give 1.876 g of (E)-3-(2-chlorophenyl)-2-(3-pyridyl)acrylic acid.

In the same manner as in Reference Example 1, 4, 7 or 8, compounds of the following Reference Examples 9 to 40 were obtained.

### Reference Example 9:

Methyl (E)-3-(3-methoxyphenyl)-2-(1-methylpyrrol-2-yl)acrylate

### Reference Example 10:

(E)-3-(3-chlorophenyl)-2-(3-pyridyl)acrylic acid

### Reference Example 11:

(E)-3-(4-chlorophenyl)-2-(3-pyridyl)acrylic acid

### Reference Example 12:

(E)-3-(2-fluorophenyl)-2-(3-pyridyl)acrylic acid

### Reference Example 13:

(E)-3-(4-fluorophenyl)-2-(3-pyridyl)acrylic acid

### Reference Example 14:

(E)-2-(3-pyridyl)-3-(2-trifluoromethylphenyl)acrylic acid

### Reference Example 15:

(E)-2-(3-pyridyl)-3-(3-trifluoromethylphenyl)acrylic acid

### Reference Example 16:

(E)-3-(2-methylsulfonylphenyl)-2-(3-pyridyl)acrylic acid

### Reference Example 17:

(E)-3-(3-methylsulfonylphenyl)-2-(3-pyridyl)acrylic acid

### Reference Example 18:

(E)-3-(2-cyanophenyl)-2-(3-pyridyl)acrylic acid

### Reference Example 19:

(E)-3-(3-cyanophenyl)-2-(3-pyridyl)acrylic acid

### Reference Example 20:

(E)-3-(2-nitrophenyl)-2-(3-pyridyl)acrylic acid

### Reference Example 21:

(E)-3-(2-methylphenyl)-2-(3-pyridyl)acrylic acid

### Reference Example 22:

(E)-3-(3-acetoxyphenyl)-2-(3-pyridyl)acrylic acid

### Reference Example 23:

(E)-3-(2-methoxyphenyl)-2-(3-pyridyl)acrylic acid

### Reference Example 24:

(E)-3-(3-methoxyphenyl)-2-(3-pyridyl)acrylic acid

### Reference Example 25:

(E)-3-(4-methoxyphenyl)-2-(3-pyridyl)acrylic acid

### Reference Example 26:

(E)-3-(3-benzyloxyphenyl)-2-(3-pyridyl)acrylic acid

### Reference Example 27:

(E)-3-(3-phenoxyphenyl)-2-(3-pyridyl)acrylic acid

### Reference Example 28:

(E)-3-(biphen-2-yl)-2-(3-pyridyl)acrylic acid

### Reference Example 29:

(E)-3-(biphen-3-yl)-2-(3-pyridyl)acrylic acid

### Reference Example 30:

(E)-3-(2,3-dichlorophenyl)-2-(3-pyridyl)acrylic acid

### Reference Example 31:

(E)-3-(2,3-dimethoxyphenyl)-2-(3-pyridyl)acrylic acid

### Reference Example 32:

(E)-3-(3,5-dimethoxyphenyl)-2-(3-pyridyl)acrylic acid

### Reference Example 33:

(E)-3-(1-naphthyl)-2-(3-pyridyl)acrylic acid

### Reference Example 34:

(E)-3-(2-naphthyl)-2-(3-pyridyl)acrylic acid

### Reference Example 35:

(E)-3-[3-(3-piperidinopropoxy)phenyl]-2-(3-pyridyl)acrylic acid formate

### Reference Example 36:

(E)-3-[3-(ethoxycarbonylmethoxy)phenyl]-2-(3-pyridyl)acrylic acid

### Reference Example 37:

(E)-3-(2-chlorophenyl)-2-(2-methylpyridin-5-yl)acrylic acid

### Reference Example 38:

(E)-3-(3-methoxyphenyl)-2-(1-methylpyrrol-2-yl)acrylic acid

### Reference Example 39:

(E)-2-(2-acetylamino-1,3-thiazol-4-yl)-3-(3-methoxyphenyl)acrylic acid

### Reference Example 40:

(E)-3-phenyl-2-(3-pyridyl)acrylic acid

### Example 1:

1.594 g of 1,1'-carbonyldiimidazole was added to a solution of 854 mg of (E)-3-(3-methoxyphenyl)-2-(2-thienyl)acrylic acid in 25 ml of dimethylformamide, and stirred at 50°C for 25 minutes. A suspension containing 2209 mg of guanidine hydrochloride and 920 mg of 60% sodium hydride in 15 ml of dimethylformamide was dropwise added to the resulting solution, while cooling with ice, and then stirred at room temperature for 7 hours. Water was added to the reaction mixture, which was then extracted with ethyl acetate. The resulting extract was dried with anhydrous magnesium sulfate, then the solvent was removed through distillation under reduced pressure, and the residue was purified on silica gel column chromatography (ethyl acetate) to give 323 mg of N-[(E)-3-(3-methoxyphenyl)-2-(2-thienyl)acryloyl]guanidine.

In the same manner as in Example 1, compounds of the following Examples 2 and 3 were obtained.

### Example 2:

N-[(E)-3-(3-methoxyphenyl)-2-(3-thienyl)acryloyl]guanidine

### Example 3:

N-[(E)-3-(2-chlorophenyl)-2-(3-pyridyl)acryloyl]guanidine

### Example 4:

0.73 g of N-[(E)-3-(3-methoxyphenyl)-2-(3-thienyl)acryloyl]guanidine was dissolved in methanol, 0.23 g of methanesulfonic acid was added thereto, and thereafter methanol was removed through distillation under reduced pressure. The resulting residue was crystallized from ethyl acetate to give 0.83 g of N-[(E)-3-(3-methoxyphenyl)-2-(3-thienyl)acryloyl]guanidine methanesulfonate.

### Example 5:

0.998 ml of a solution of 1 M of methanesulfonic acid in methanol was added to a solution of 300 mg of N-[(E)-3-(2-chlorophenyl)-2-(3-pyridyl)acryloyl]guanidine in 10 ml of methanol, and stirred at room temperature for 1 minute, and then the solvent was removed through distillation under reduced pressure. The resulting residue was crystallized from ethyl acetate to give 383 mg of crude crystals. 250 mg of the crude crystals were recrystallized from ethanol to obtain 207 mg of N-[(E)-3-(2-chlorophenyl)-2-(3-pyridyl)acryloyl]guanidine methanesulfonate.

In the same manner as in Example 1, 4 or 5, compounds of the following Examples 6 to 46 were obtained.

### Example 6:

N-[(E)-3-(3-chlorophenyl)-2-(3-pyridyl)acryloyl]guanidine

### Example 7:

N-[(E)-3-(4-chlorophenyl)-2-(3-pyridyl)acryloyl]guanidine

### Example 8:

N-[(E)-3-(4-chlorophenyl)-2-(3-pyridyl)acryloyl]guanidine methanesulfonate

### Example 9:

N-[(E)-3-(2-fluorophenyl)-2-(3-pyridyl)acryloyl]guanidine

### Example 10:

N-[(E)-3-(2-fluorophenyl)-2-(3-pyridyl)acryloyl]guanidine methanesulfonate

### Example 11:

N-[(E)-3-(4-fluorophenyl)-2-(3-pyridyl)acryloyl]guanidine

### Example 12:

N-[(E)-3-(3-pyridyl)-3-(2-trifluoromethylphenyl)acryloyl]guanidine

### Example 13:

N-[(E)-2-(3-pyridyl)-3-(3-trifluoromethylphenyl)acryloyl]guanidine

### Example 14:

N-[(E)-3-(2-methylsulfonylphenyl)-2-(3-pyridyl)acryloyl]guanidine

### Example 15:

N-[(E)-3-(3-methylsulfonylphenyl)-2-(3-pyridyl)acryloyl]guanidine

### Example 16:

N-[(E)-3-(2-cyanophenyl)-2-(3-pyridyl)acryloyl]guanidine

### Example 17:

N-[(E)-3-(3-cyanophenyl)-2-(3-pyridyl)acryloyl]guanidine

### Example 18:

N-[(E)-3-(2-nitrophenyl)-2-(3-pyridyl)acryloyl]guanidine

### Example 19:

N-[(E)-3-(2-methylphenyl)-2-(3-pyridyl)acryloyl]guanidine

### Example 20:

N-[(E)-3-(2-methylphenyl)-2-(3-pyridyl)acryloyl]guanidine methanesulfonate

### Example 21:

N-[(E)-3-(3-hydroxyphenyl)-2-(3-pyridyl)acryloyl]guanidine

### Example 22:

N-[(E)-3-(3-hydroxyphenyl)-2-(3-pyridyl)acryloyl]guanidine methanesulfonate

### Example 23:

N-[(E)-3-(2-methoxyphenyl)-2-(3-pyridyl)acryloyl]guanidine

### Example 24:

N-[(E)-3-(2-methoxyphenyl)-2-(3-pyridyl)acryloyl]guanidine methanesulfonate

### Example 25:

N-[(E)-3-(3-methoxyphenyl)-2-(3-pyridyl)acryloyl]guanidine

### Example 26:

N-{(E)-3-(3-methoxyphenyl)-2-(3-pyridyl)acryloyl]guanidine methanesulfonate

### Example 27:

N-[(E)-3-(4-methoxyphenyl)-2-(3-pyridyl)acryloyl]guanidine

### Example 28:

N-[(E)-3-(4-methoxyphenyl)-2-(3-pyridyl)acryloyl]guanidine methanesulfonate

### Example 29:

N-[(E)-3-(3-benzyloxyphenyl)-2-(3-pyridyl)acryloyl]guanidine

### Example 30:

N-[(E)-3-(3-benzyloxyphenyl)-2-(3-pyridyl)acryloyl]guanidine methanesulfonate

### Example 31:

N-[(E)-3-(3-phenoxyphenyl)-2-(3-pyridyl)acryloyl]guanidine

### Example 32:

N-[(E)-3-(biphen-2-yl)-2-(3-pyridyl)acryloyl]guanidine methanesulfonate

### Example 33:

N-[(E)-3-(biphen-3-yl)-2-(3-pyridyl)acryloyl]guanidine 1.35methanesulfonate

### Example 34:

N-[(E)-3-(2,3-dichlorophenyl)-2-(3-pyridyl)acryloyl]guanidine

### Example 35:

N-[(E)-3-(2,3-dimethoxyphenyl)-2-(3-pyridyl)acryloyl]guanidine

### Example 36:

N-[(E)-3-(3,5-dimethoxyphenyl)-2-(3-pyridyl)acryloyl]guanidine

### Example 37:

N-[(E)-3-(1-naphthyl)-2-(3-pyridyl)acryloyl]guanidine 1.1methanesulfonate

### Example 38:

N-[(E)-3-(2-naphthyl)-2-(3-pyridyl)acryloyl]guanidine methanesulfonate

### Example 39:

N-[(E)-3-[3-(3-piperidinopropoxy)phenyl]-2-(3-pyridyl)acryloyl]guanidine

### Example 40:

N-[(E)-3-[3-(ethoxycarbonylmethoxy)phenyl]-2-(3-pyridyl)acryloyl]guanidine 2hydrochloride

### Example 41:

N-[(E)-3-(2-chlorophenyl)-2-(2-methylpyridin-5-yl)acryloyl]guanidine

### Example 42:

N-[(E)-3-(2-chlorophenyl)-2-(2-methylpyridin-5-yl)acryloyl]guanidine methanesulfonate

### Example 43:

N-[(E)-3-(3-methoxyphenyl)-2-(1-methylpyrrol-2-yl)acryloyl]guanidine

### Example 44:

N-[(E)-2-(2-acetylamino-1,3-thiazol-4-yl)-3-(3-methoxyphenyl)acryloyl]guanidine

### Example 45:

N-[(E)-3-phenyl-2-(3-pyridyl)acryloyl]guanidine

### Example 46:

N-[(E)-3-phenyl-2-(3-pyridyl)acryloyl]guanidine methanesulfonate

### Example 47:

A mixture comprised of 408 mg of N-[(E)-3-(2-nitrophenyl)-2-(3-pyridyl)acryloyl]guanidine, 391 mg of tin and 14 ml of an aqueous solution of 10 % hydrochloric acid was stirred at 100°C for 27 minutes, and then left cooled at room temperature. A solution of 1N sodium hydroxide was added thereto, in which the white precipitate formed was dissolved. This was thereafter extracted with a mixed solution of chloroform/isopropanol (= 3/1). The resulting extract was dried with magnesium sulfate, the solvent was removed through distillation under reduced pressure, and the residue was purified on column chromatography to give 251 mg of N-[(E)-3-(2-aminophenyl)-2-(3-pyridyl)acryloyl]guanidine.

The structural formulae and the physico-chemical properties of the compounds obtained in the above-mentioned reference examples and examples are shown in the following tables, in which the abbreviations used have the following meanings.
- Rf.:: Number of Reference Example
- Ex.:: Number of Example
- Sal:: Salt
- mp.:: Melting point
- NMR:: Nuclear magnetic resonance (δ, DMSO-d6, TMS internal standard, unless otherwise specifically indicated)
- m/z:: Mass spectrum (m/z)
- Bn:: Benzyl
- Ms:: Methylsulfonyl
- MsOH:: Methanesulfonic acid
- Naph:: Naphthyl
- Ph:: Phenyl
- Py:: Pyridyl
- Th:: Thienyl
- 1-Me-Pyr:: 1-Methylpyrrolyl
- Ac:: Acetyl
- AcNH:: Acetylamino
- Thi:: 1,3-Thiazolyl
- 3-Pi-Pro:: 3-Piperidinopropoxy
- EtO-CO-MeO:: Ethoxycarbonylmethoxy

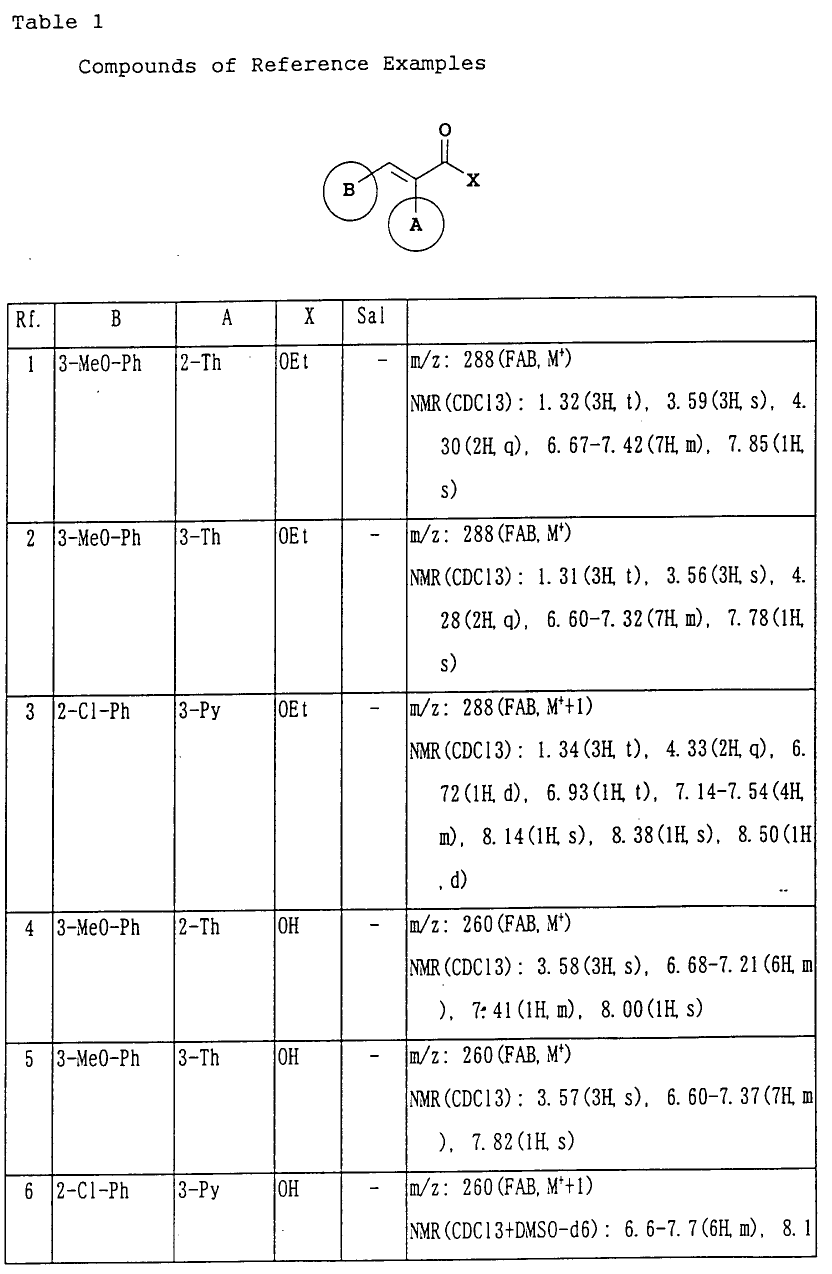

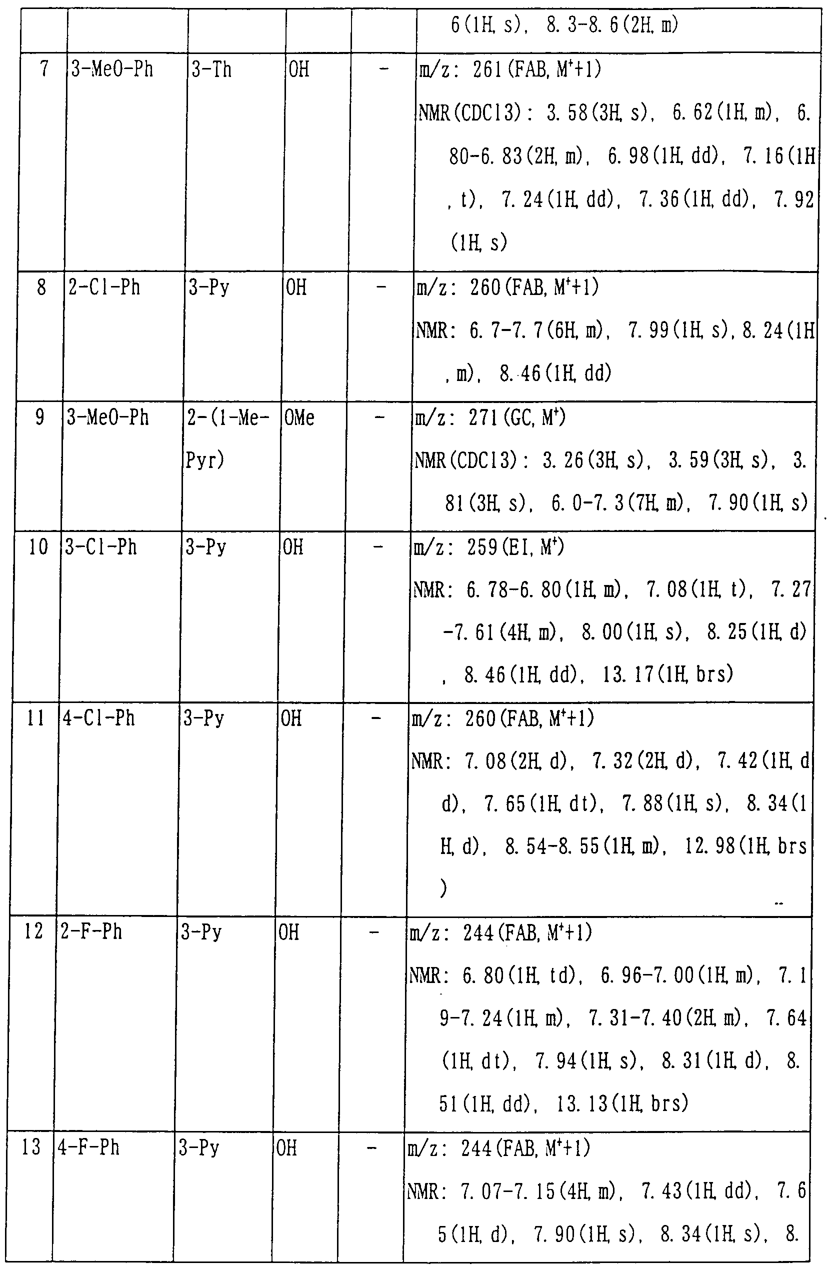

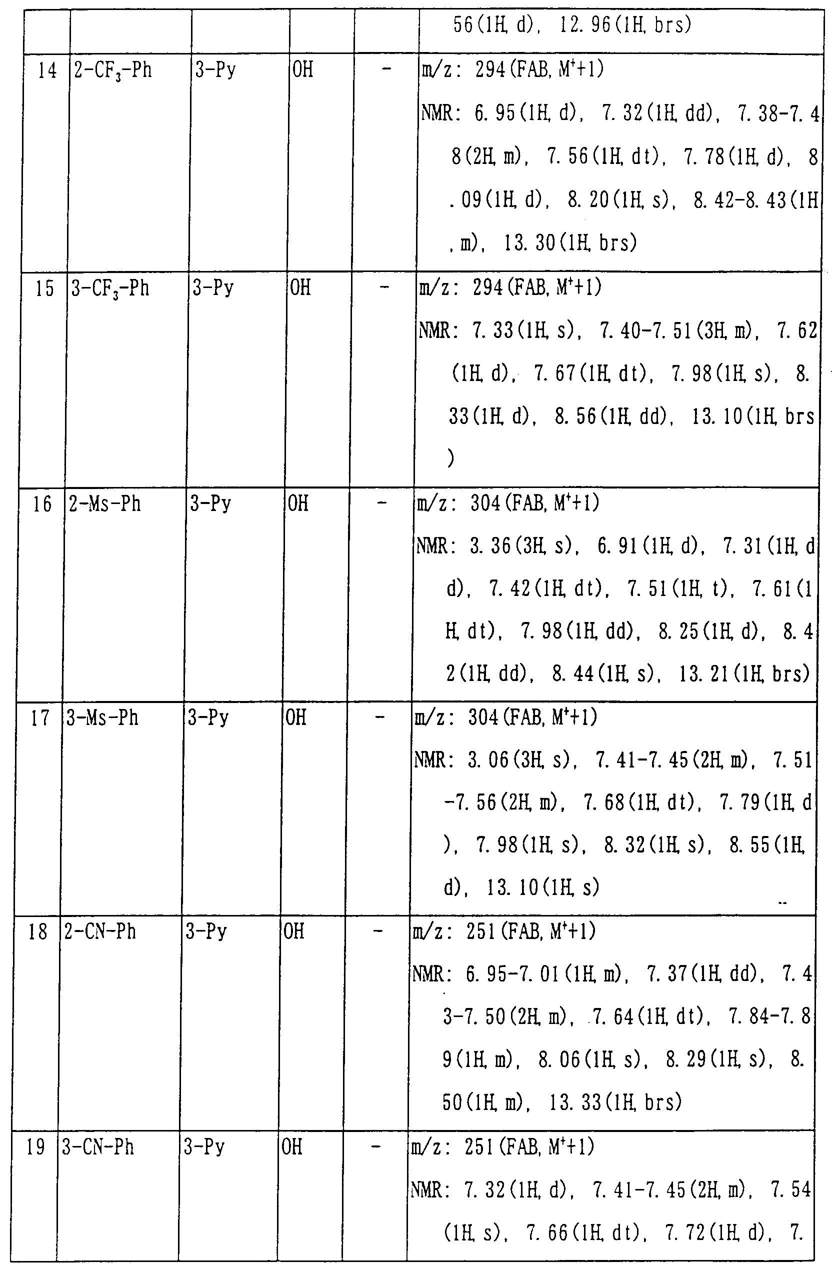

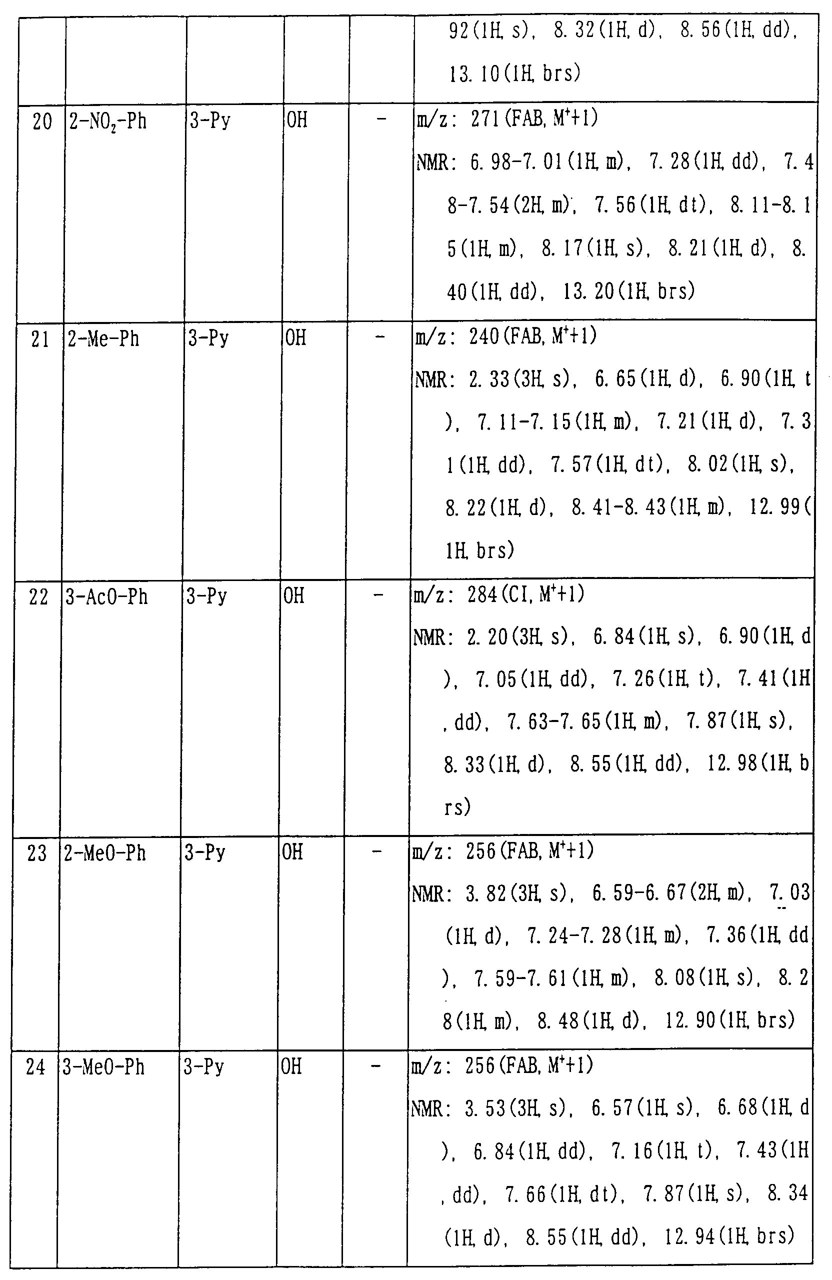

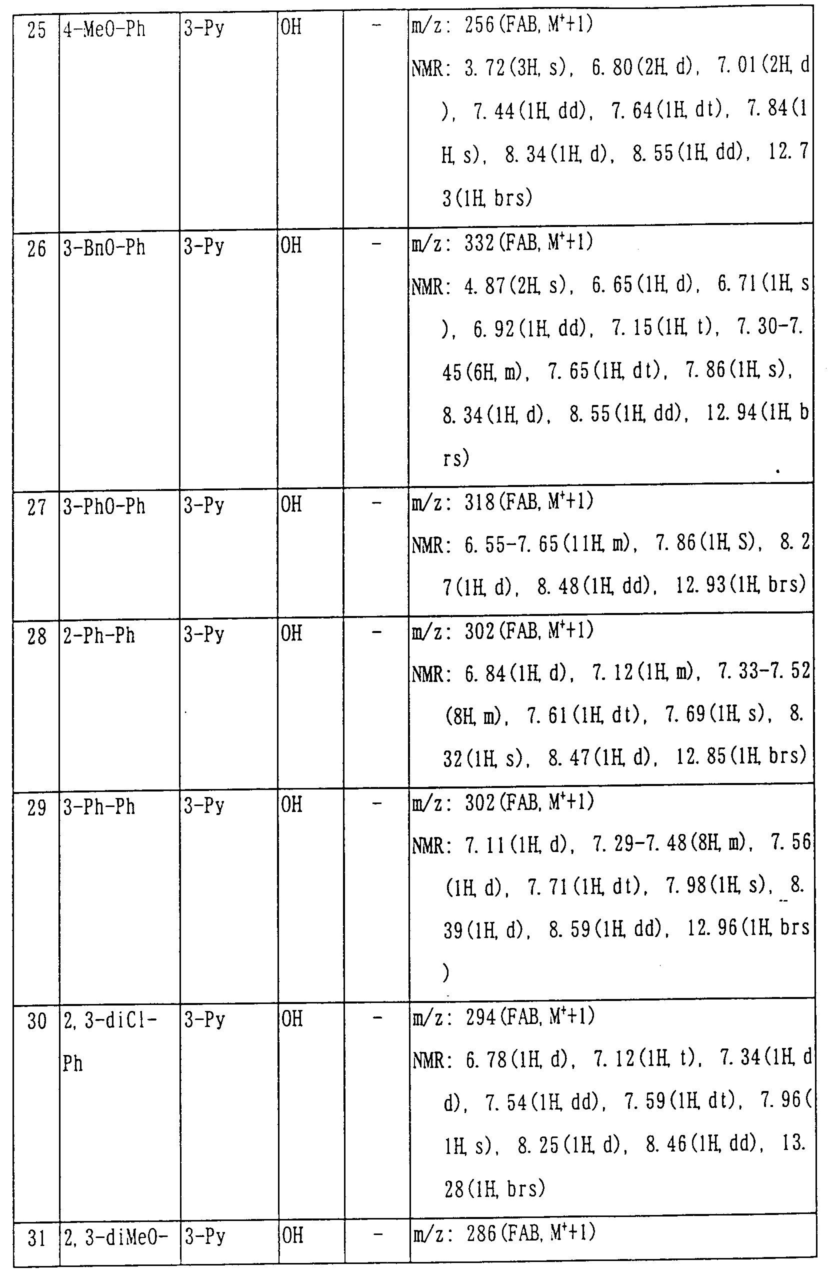

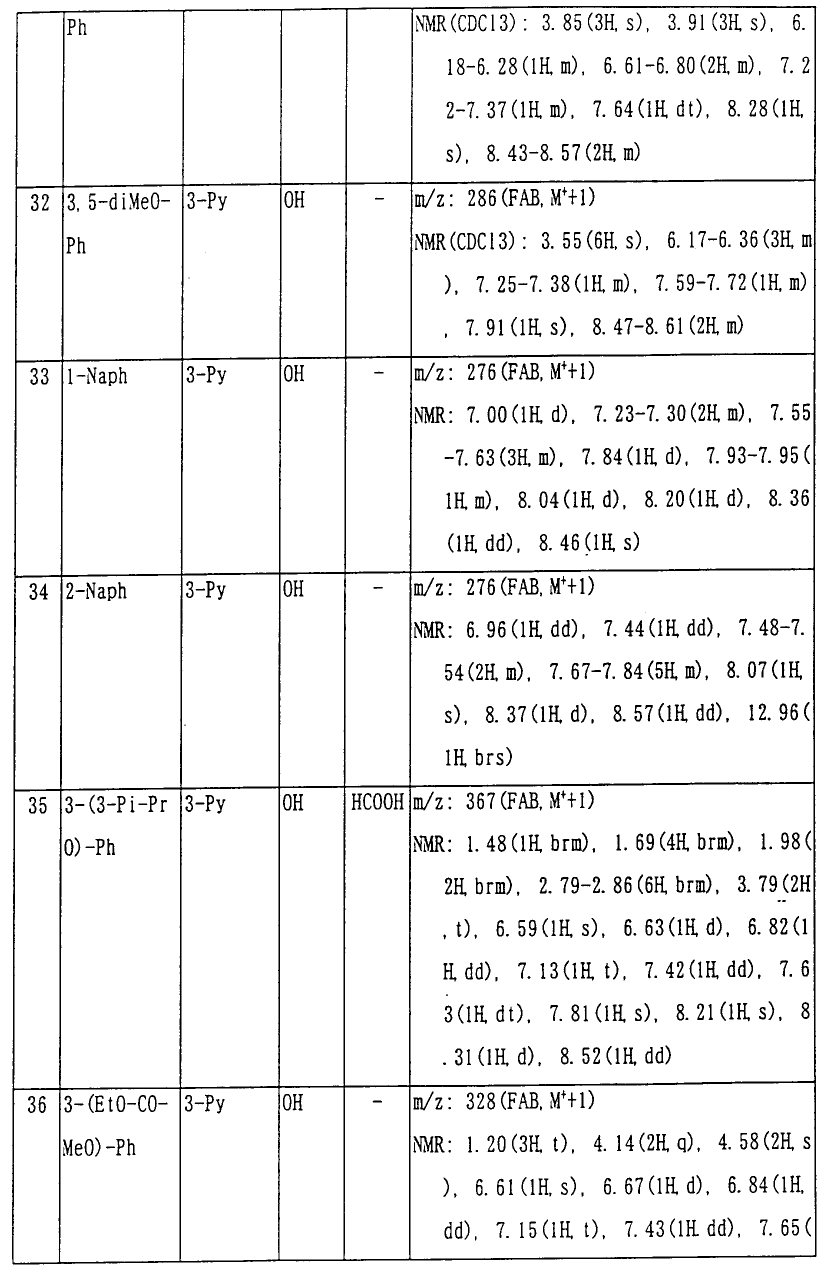

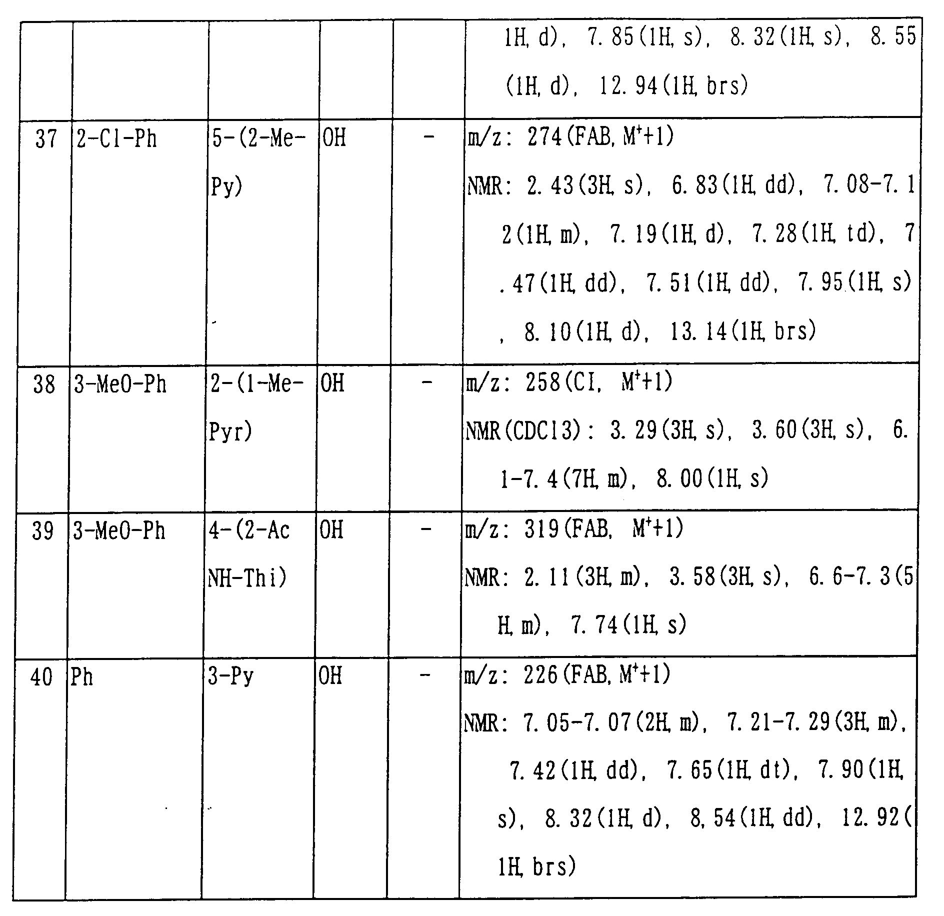

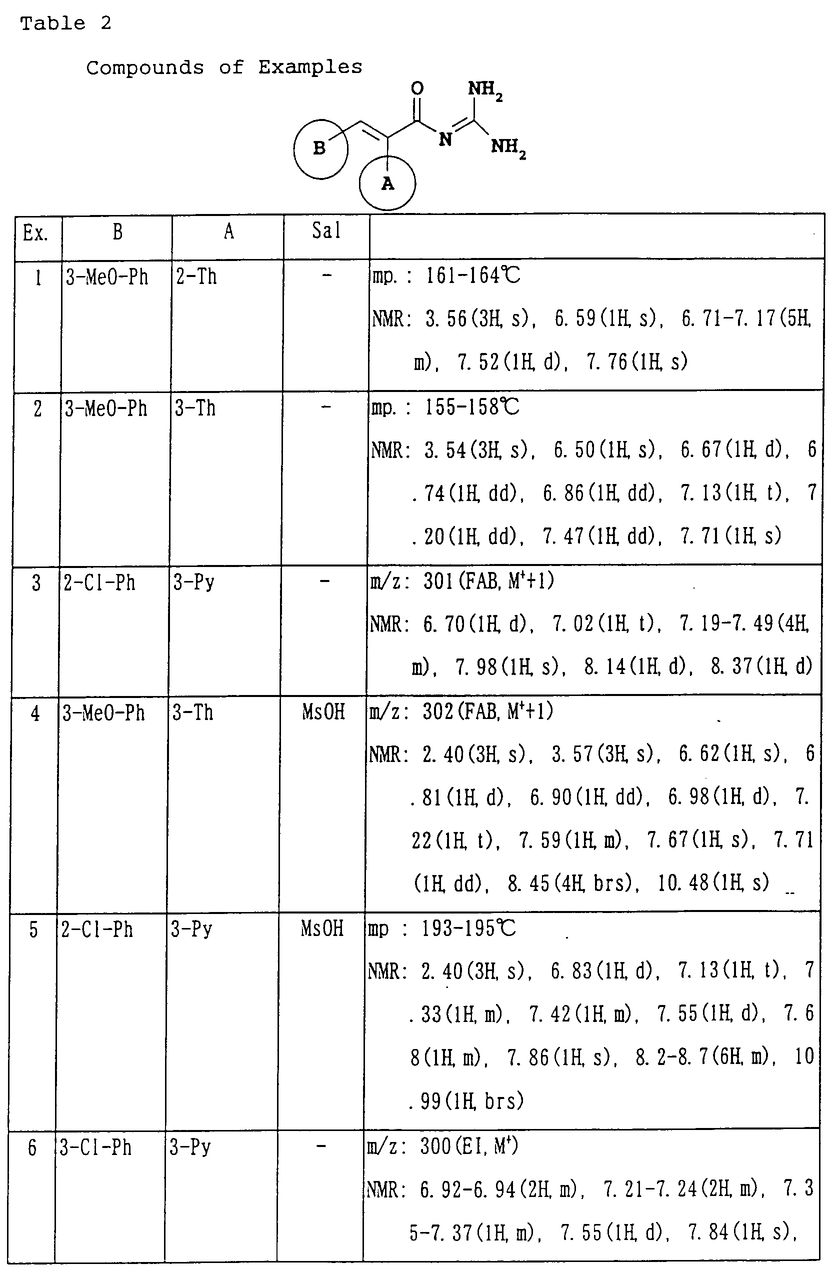

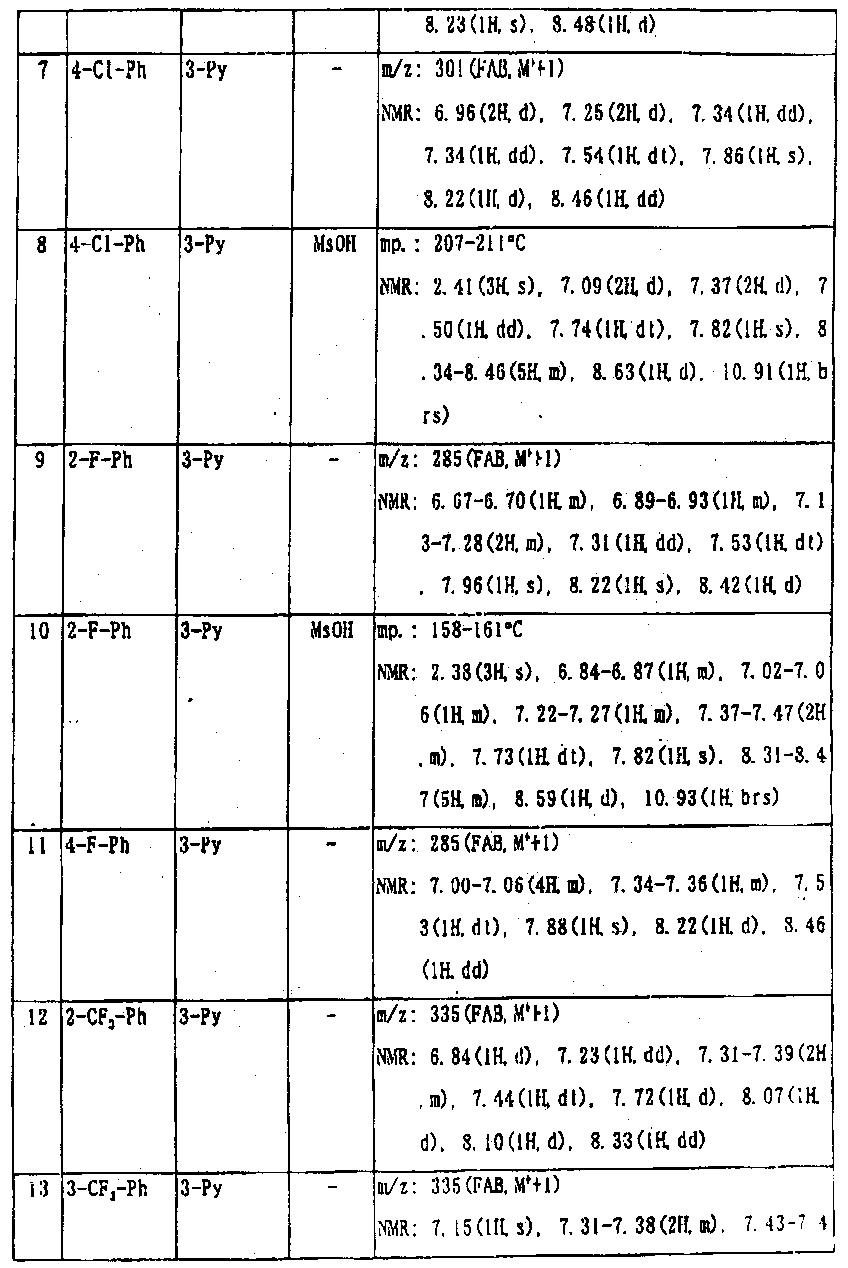

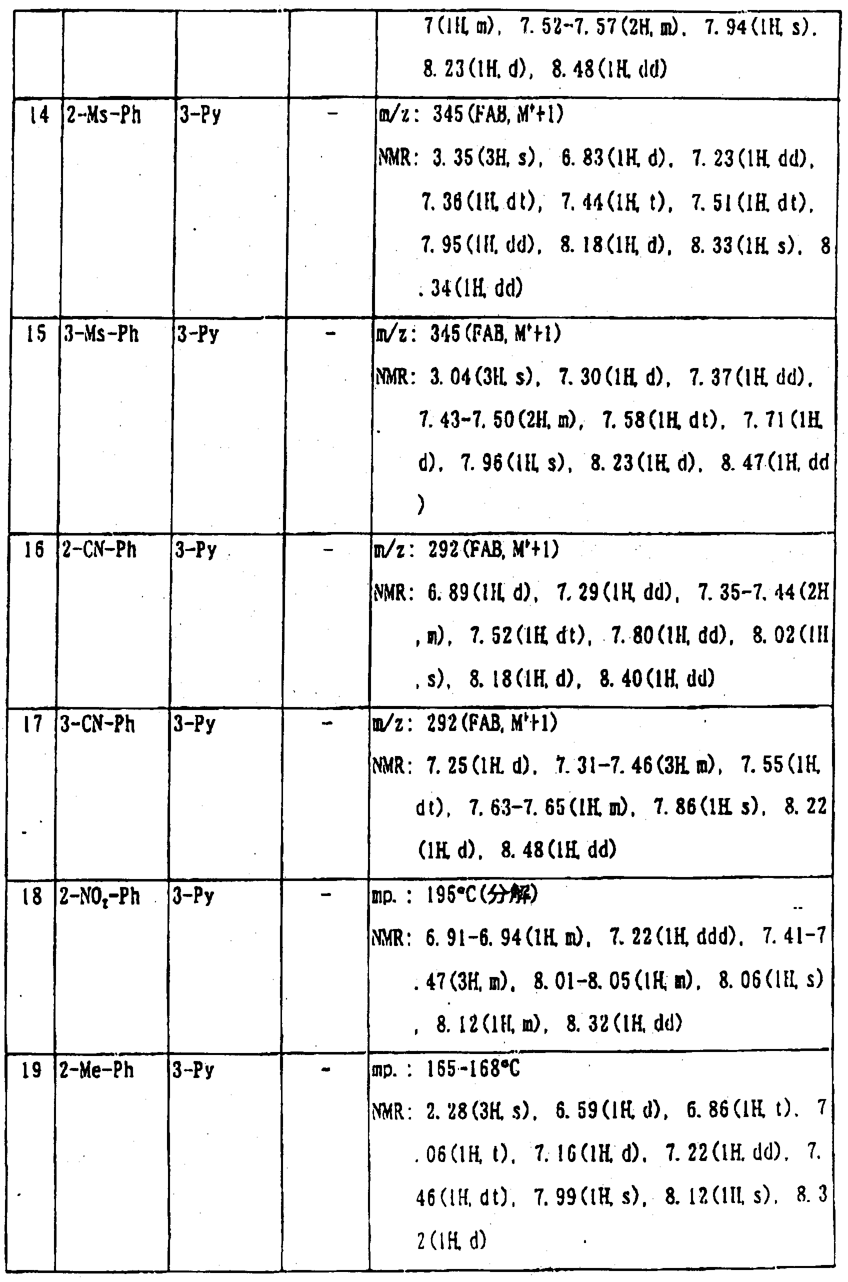

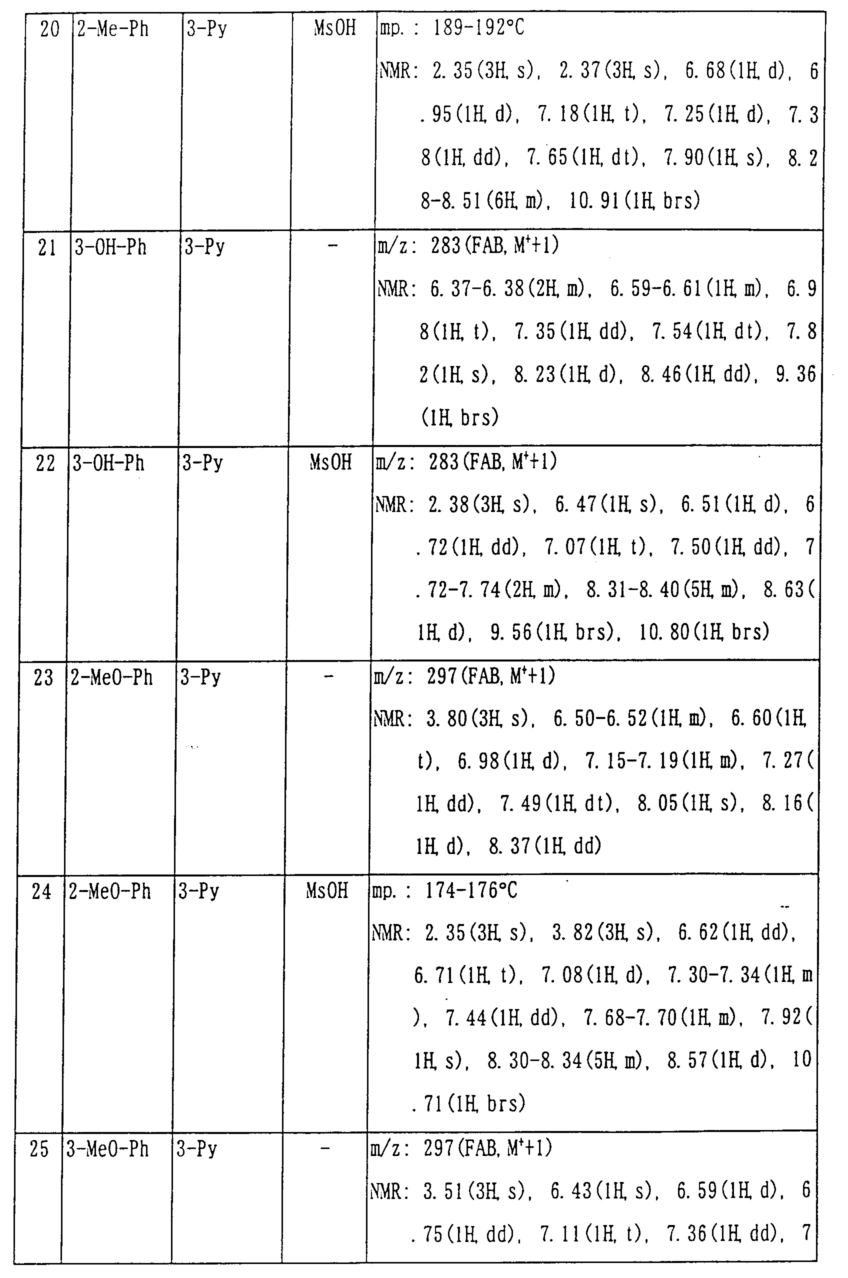

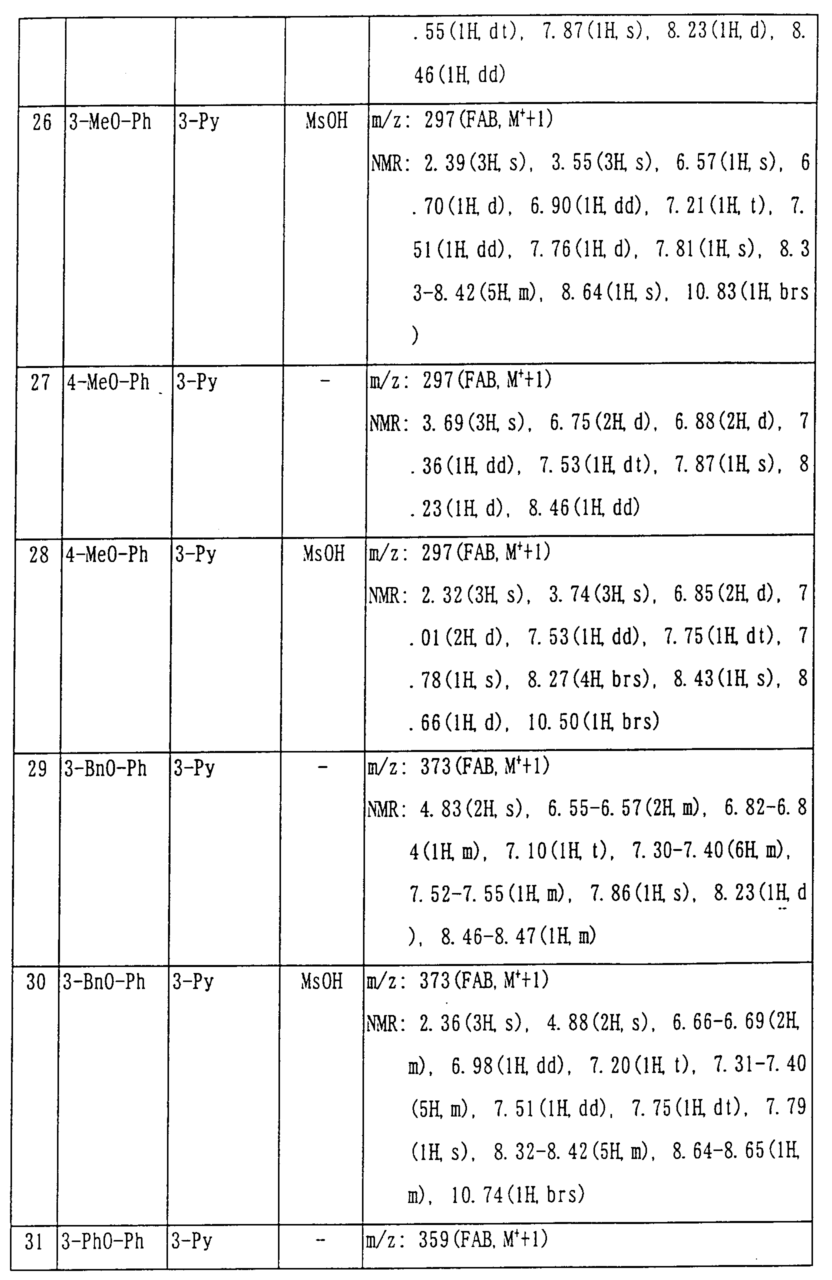

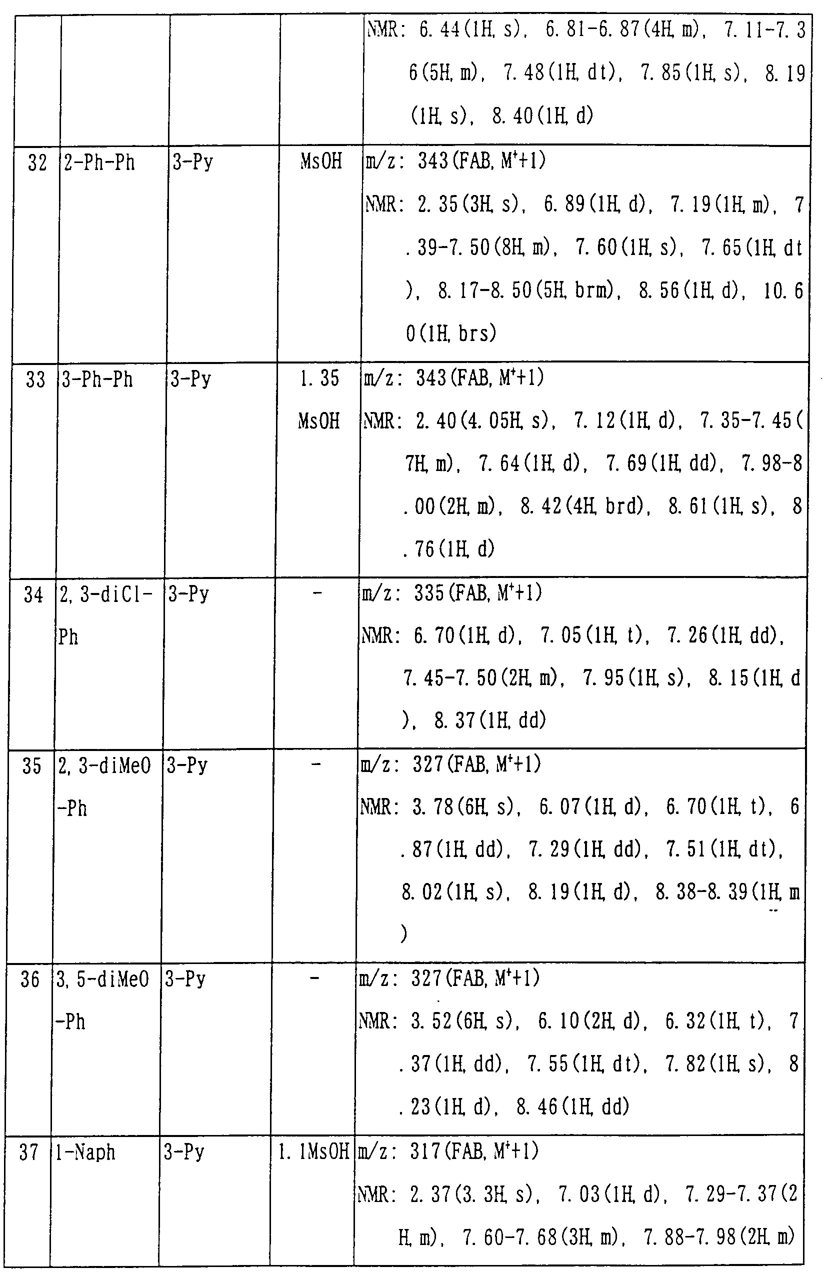

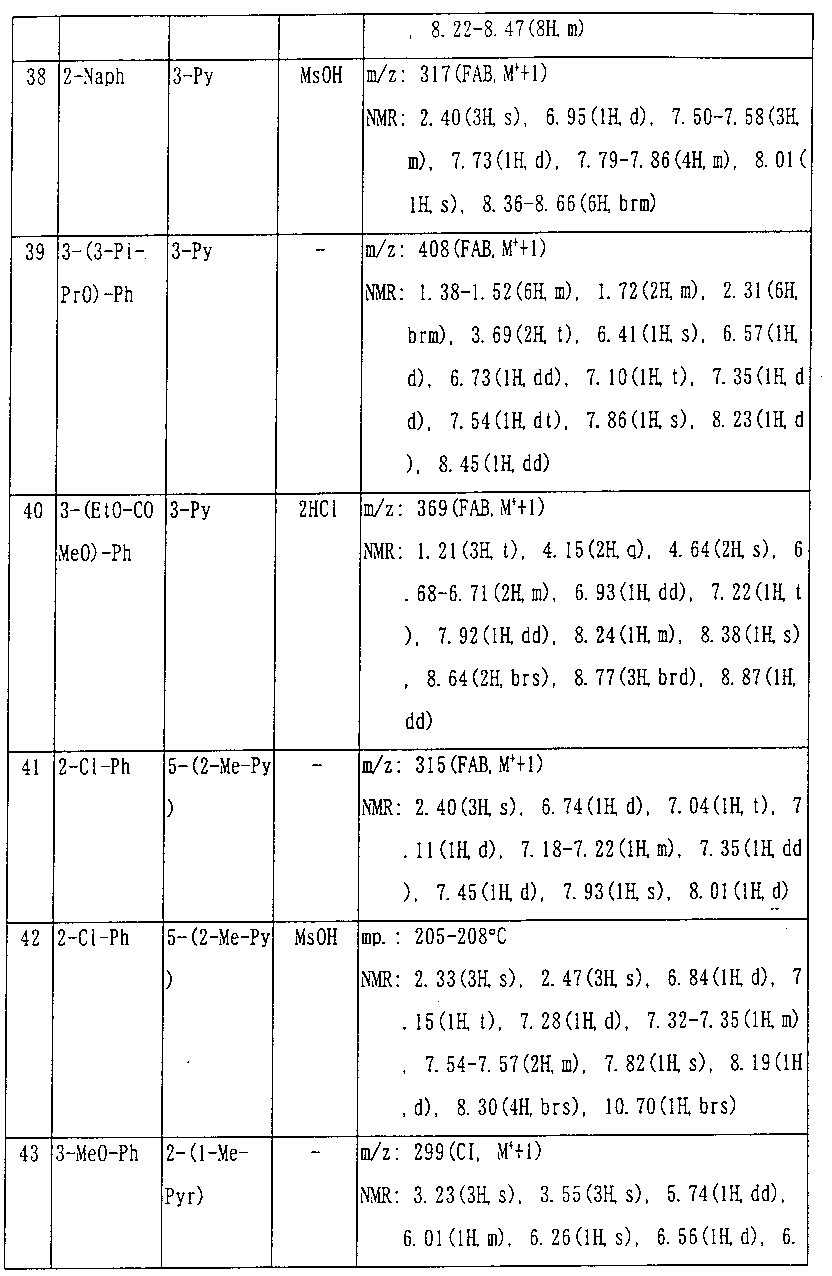

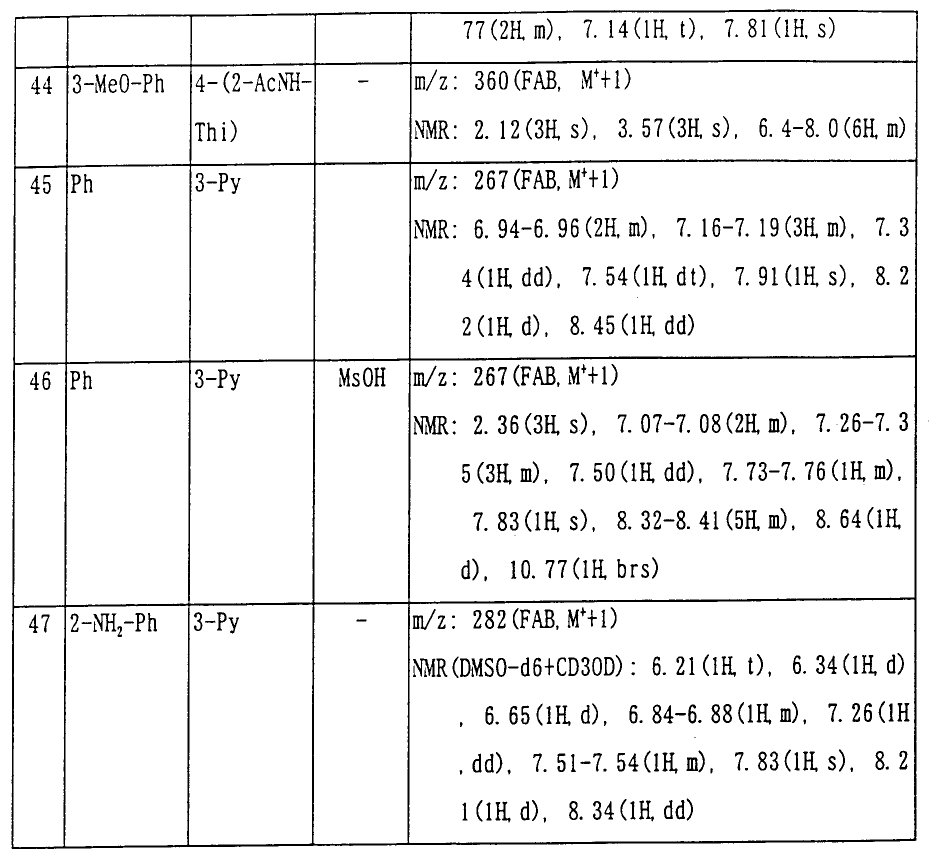

The compounds of which chemical structures described in the following Table 3 and 4 can be synthesized by any one of the above-described preparation processes, preparation pathways and methods described in Examples or processes known to those skilled in the art and do not require any particular experiment.

The abbreviations used in the tables have the same meanings as those mentioned hereinabove, and Co. represents Number of Compound.

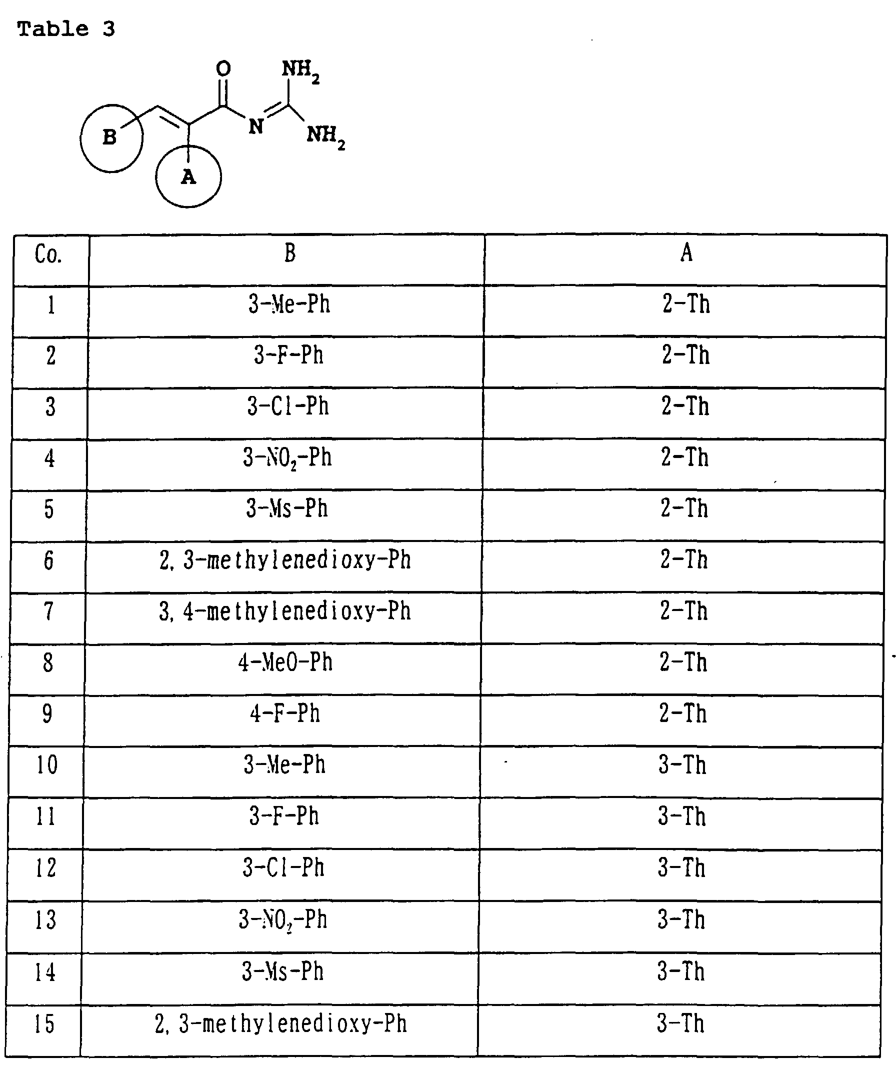

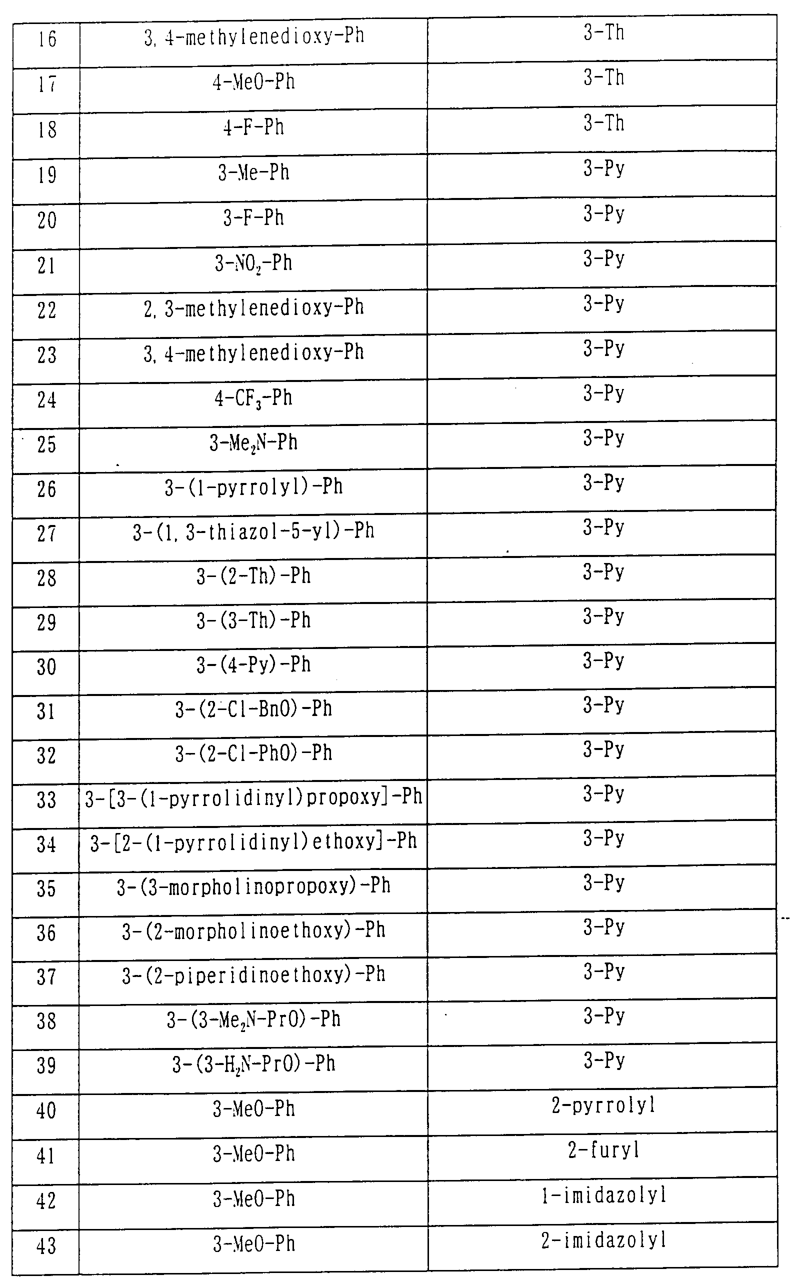

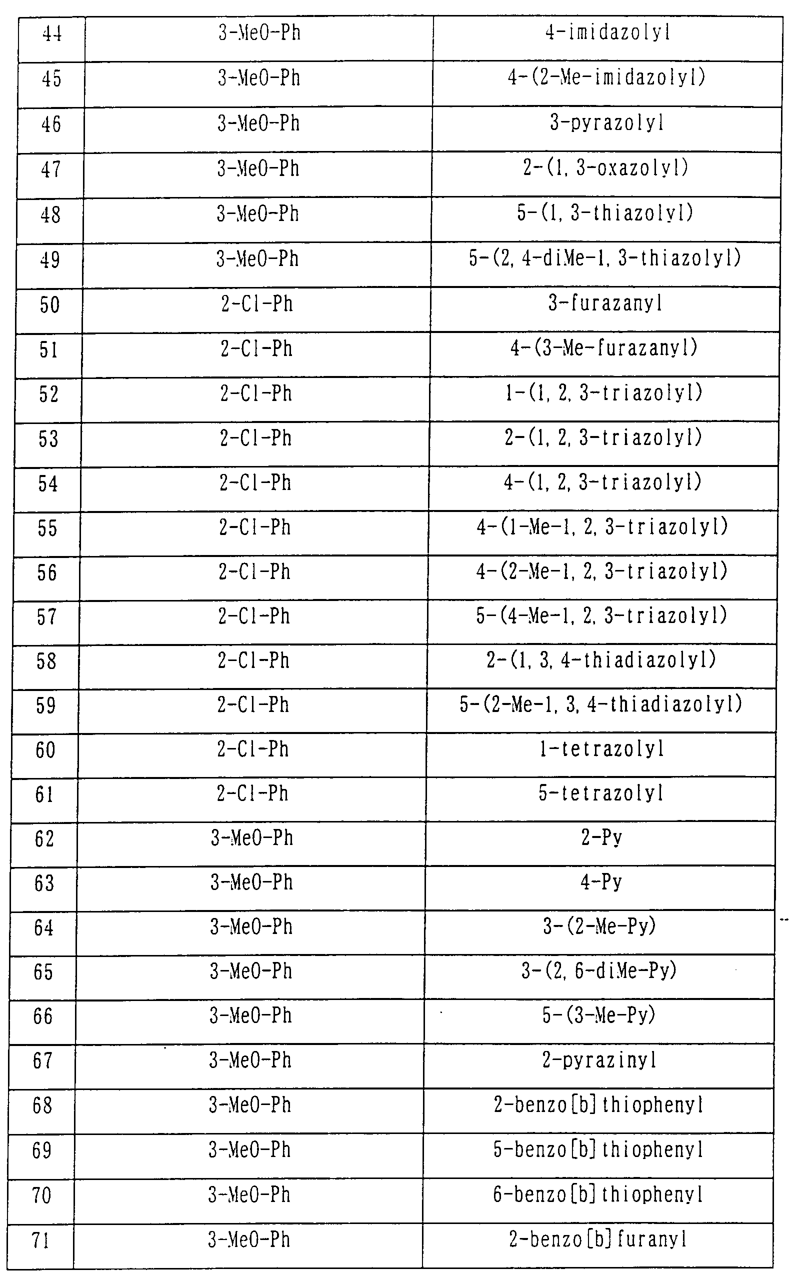

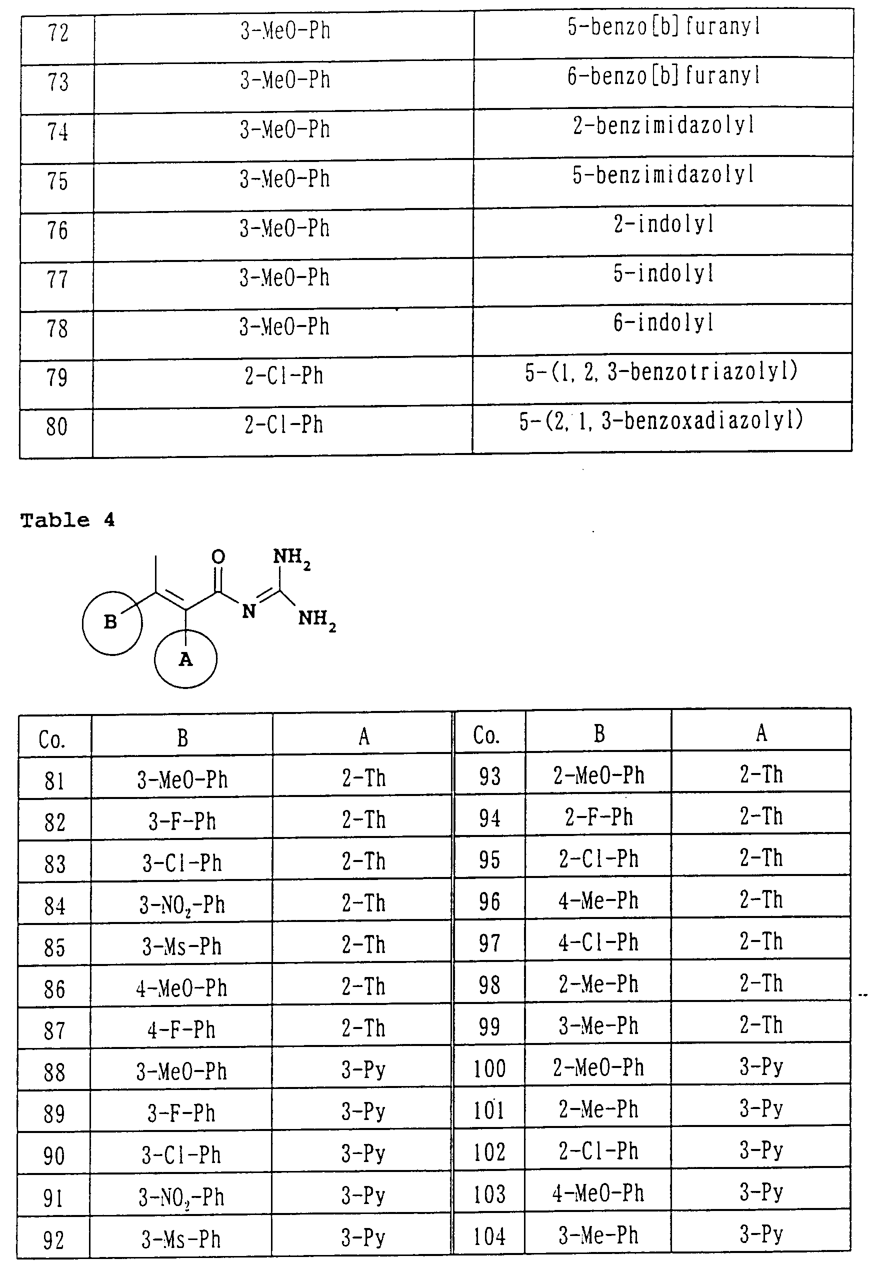

## Claims

1. Heteroaryl-substituted acryloylguanidine derivatives of the following general formula (I) and their salts. 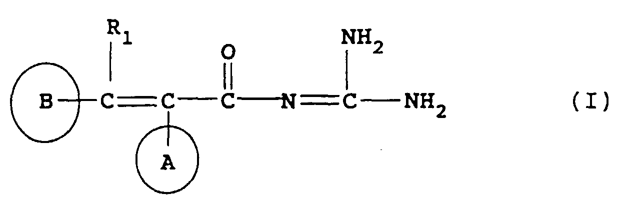 wherein ring A represents a 5-membered or 6-membered heteroaryl group which may optionally be substituted and may optionally be condensed with one or two benzene rings; ring B represents an optionally-substituted aryl group; and R₁ represents a hydrogen atom, a halogen atom, or a lower alkyl group optionally substituted by one or more halogen atoms.

2. Heteroaryl-substituted acryloylguanidine derivatives and their salts as claimed in claim 1, wherein the ring A is a 5-membered or 6-membered heteroaryl group which may optionally be substituted by one or more substituents selected from the members of the following group D and which may optionally be condensed with one or two benzene rings, and the ring B is an aryl group which may optionally be substituted by one or more substituents selected from the members of the following group E. Group D, Group E: A lower alkyl group optionally substituted by one or more halogen atoms, a lower alkenyl group, a lower alkynyl group, a cycloalkyl group, a lower alkoxy group, a lower alkoxy-lower alkoxy group, a lower alkoxycarbonyl-lower alkoxy group, a carboxyl-lower alkoxy group, an amino-lower alkoxy group, a mono- or di-lower alkylamino-lower alkoxy group, a nitrogen-containing, saturated heterocyclic group-substituted lower alkoxy group, a lower alkoxycarbonyl group, a carboxyl group, a halogen atom, a nitro group, a cyano group, an amino group, a mono- or di-lower alkylamino group, a lower alkanoyl group, a lower alkanoylamino group, a lower alkanoyl-lower alkylamino group, a lower alkanoyloxy group, a hydroxyl group, a mercapto group, a lower alkylthio group, a lower alkylsulfinyl group, a lower alkylsulfonyl group, an aminosulfonyl group, a mono- or di-lower alkylaminosulfonyl group, a lower alkylsulfonylamino group, a lower alkylsulfonyl-mono-lower alkylamino group, a carbamoyl group, a mono- or di-lower alkylaminocarbonyl group, a methylenedioxy group, an ethylenedioxy group, a propylenedioxy group, an optionally-substituted aryl group, an optionally-substituted aralkyl group, an optionally-substituted, 5-membered or 6-membered heteroaryl group, an optionally-substituted aryloxy group, and an optionally-substituted aralkyloxy group.

3. Heteroaryl-substituted acryloylguanidine derivatives and their salts as claimed in claim 2, wherein the group D consists of a lower alkyl group optionally substituted by one or more halogen atoms, a lower alkenyl group, a lower alkynyl group, a lower alkoxy group, a halogen atom, a nitro group, a cyano group, an amino group, a mono- or di-lower alkylamino group, a lower alkanoyl group, a lower alkanoylamino group, a lower alkanoyl-lower alkylamino group, a lower alkanoyloxy group, a hydroxyl group, a mercapto group, a lower alkylthio group, a lower alkylsulfinyl group, a lower alkylsulfonyl group, an aminosulfonyl group, a mono- or di-lower alkylaminosulfonyl group, a lower alkylsulfonylamino group, a lower alkylsulfonyl-mono-lower alkylamino group, a carbamoyl group, and a mono- or di-lower alkylaminocarbonyl group, and the group E consists of a lower alkyl group optionally substituted by one or more halogen atoms, a lower alkenyl group, a lower alkynyl group, a cycloalkyl group, a lower alkoxy group, a lower alkoxy-lower alkoxy group, a lower alkoxycarbonyl-lower alkoxy group, a carboxy-lower alkoxy group, an amino-lower alkoxy group, a mono- or di-lower alkylamino-lower alkoxy group, a nitrogen-containing, saturated heterocyclic group-substituted lower alkoxy group, a lower alkoxycarbonyl group, a carboxyl group, a halogen atom, a nitro group, a cyano group, an amino group, a mono- or di-lower alkylamino group, a lower alkanoyl group, a lower alkanoylamino group, a lower alkanoyl-lower alkylamino group, a lower alkanoyloxy group, a hydroxyl group, a mercapto group, a lower alkylthio group, a lower alkylsulfinyl group, a lower alkylsulfonyl group, an aminosulfonyl group, a mono- or di-lower alkylaminosulfonyl group, a lower alkylsulfonylamino group, a lower alkylsulfonyl-mono-lower alkylamino groups, a carbamoyl group, a mono- or di-lower alkylaminocarbonyl group, a methylenedioxy group, an ethylenedioxy group, a propylenedioxy group, an aryl group, an aralkyl group, a 5-membered or 6-membered heteroaryl group, an aryloxy group, and an aralkyloxy group.

4. Heteroaryl-substituted acryloylguanidine derivatives and their salts as claimed in claim 3, wherein R₁ is a hydrogen atom.

5. Heteroaryl-substituted acryloylguanidine derivatives and their salts as claimed in claim 4, wherein the ring A is a 5-membered or 6-membered heteroaryl group which may optionally be substituted by one or more substituents selected from a lower alkyl group and a lower alkanoylamino group and which may optionally be condensed with one or two benzene rings; and the ring B is a phenyl group which may optionally be substituted by one or more substituents selected from a lower alkyl group optionally substituted by one or more halogen atoms, a lower alkoxy group, a lower alkoxycarbonyl-lower alkoxy group, a nitrogen-containing, saturated heterocyclic group-substituted lower alkoxy group, a halogen atom, a nitro group, a cyano group, an amino group, a hydroxyl group, a lower alkylsulfonyl group, an aryl group, an aryloxy group and an aralkyloxy group, or is an unsubstituted naphthyl group.

6. Heteroaryl-substituted acryloylguanidine derivatives and their salts as claimed in any one of claims 2 to 5, wherein the ring A is a 5-membered or 6-membered heteroaryl group selected from a pyridyl group, a thienyl group, a thiazolyl group, a pyrrolyl group, a benzimidazolyl group and a pyrazinyl group.

7. Heteroaryl-substituted acryloylguanidine derivatives and their salts as claimed in any one of claims 1 to 6, wherein the ring A and the ring B are cis-configured.

8. A pharmaceutical composition comprising a heteroaryl-substituted acryloylguanidine derivative or its salt as claimed in claim 1, and a pharmaceutically-acceptable carrier.

9. The pharmaceutical composition as claimed in claim 8, which is an Na⁺/H⁺ exchange inhibitor.

10. The pharmaceutical composition as claimed in claim 9, which is used in prevention, treatment and diagnosis of disorders to be caused by the promotion of the activities of the Na⁺/H⁺ exchange, such as hypertension, arrhythmia, angina pectoris, cardiac hypertrophy, organopathy to be induced by ischemic-reperfusion, disorders to be induced by the depression of blood flow in organo-transplantation, myocardial infarction, recurrence of myocardial infarction, ischemic organopathy, cell-proliferative disorders, arteriosclerosis, diabetic complications, cancer, vascular hypertrophy, tylosis and hypertrophy of tissues and organs, cardiofibrosis, pulmofibrosis, hepatofibrosis, renofibrosis, renal glomerulosclerosis, protection of transplants, edema, cerebredema, chronic cardiopathy, cardiomyopathy, pulmonary embolism, acute and chronic nephropathy, cerebral infarction, chronic cerebrocirculation disorders, cranial nerves disorders, disorders caused by hyperglycemia, disorders as associated with insulin resistance, shocks, inflammatory disorders, pneumonopathy, bronchopathy, osteoporosis, and acid-base balance disorders.
